# EUROPEAN PATENT SPECIFICATION

(11) **EP 4 263 719 B1**
(45) Date of publication and mention of the grant of the patent: **12.08.2026**
(21) Application number: 21823951.5
(22) Date of filing: 16.12.2021
(51) Int. Cl.: C09B 23/01, G01N 33/84, C09B 23/08, G01N 33/58

(54) **PH RESPONSIVE CYANINE DYES AND CONJUGATES THEREOF**
PH-REAKTIVE CYANINFARBSTOFFE UND KONJUGATE DAVON
COLORANTS DE CYANINE SENSIBLES AU PH ET LEURS CONJUGUÉS

(30) Priority: 17.12.2020 EP 20214798
(43) Date of publication of application: 25.10.2023
(73) Proprietor: Bracco Imaging SpA, 20134 Milano (IT)
(72) Inventor: NAPOLITANO, Roberta, 10010 Colleretto Giacosa (IT); BLASI, Francesco, 10010 Colleretto Giacosa (IT); FERRETTI, Fulvio, 10010 Colleretto Giacosa (IT); DIALE, Nicolò, 10010 Colleretto Giacosa (IT); BUONSANTI, Federica, 10010 Colleretto Giacosa (IT); VISIGALLI, Massimo, 10010 Colleretto Giacosa (IT); ADAMO, Alessia, 10010 Colleretto Giacosa (IT)
(74) Representative: Poletti, Marco
(86) International application number: PCT/EP2021/086202
(87) International publication number: WO 2022/129336

(56) References cited:
- CN-A- 105 693 590
- WYCISK VIRGINIA ET AL: "Responsive Contrast Agents: Synthesis and Characterization of a Tunable Series of pH-Sensitive Near-Infrared Pentamethines", vol. 1, no. 5, 4 November 2016 (2016-11-04), US, pages 808 - 817, XP055812962, ISSN: 2470-1343, Retrieved from the Internet <URL:https://pubs.acs.org/doi/pdf/10.1021/acsomega.6b00182> DOI: 10.1021/acsomega.6b00182
- DONSKYI IEVGEN S. ET AL: "Fullerene Polyglycerol Amphiphiles as Unimolecular Transporters", vol. 33, no. 26, 7 April 2017 (2017-04-07), US, pages 6595 - 6600, XP055812984, ISSN: 0743-7463, Retrieved from the Internet <URL:https://pubs.acs.org/doi/pdf/10.1021/acs.langmuir.7b00183> DOI: 10.1021/acs.langmuir.7b00183
- XUE FENGFENG ET AL: "A smart drug: a pH-responsive photothermal ablation agent for Golgi apparatus activated cancer therapy", vol. 53, no. 48, 1 January 2017 (2017-01-01), pages 6424 - 6427, XP055812946, ISSN: 1359-7345, Retrieved from the Internet <URL:https://pubs.rsc.org/en/content/articlepdf/2017/cc/c7cc03168h> DOI: 10.1039/C7CC03168H
- LEE HYERAN ET AL: "Near-Infrared pH-Activatable Fluorescent Probes for Imaging Primary and Metastatic Breast Tumors", BIOCONJUGATE CHEMISTRY, vol. 22, no. 4, 9 March 2011 (2011-03-09), US, pages 777 - 784, XP055812917, ISSN: 1043-1802, Retrieved from the Internet <URL:https://pubs.acs.org/doi/pdf/10.1021/bc100584d> DOI: 10.1021/bc100584d

## Description

### FIELD OF THE INVENTION

The present invention relates to the field of optical imaging. More particularly, it relates to compounds of the family of monoalkylated cyanine dyes with red to near-infrared emission (650-900 nm), whose fluorescence is responsive to pH, and to conjugates with biological ligands thereof. The invention further relates to the use of these compounds as diagnostic agents, to the methods for their preparation and to the compositions comprising them.

### BACKGROUND ART

Dyes are chemical entities that absorb photons of a specific wavelength upon light excitation and re-emit some of that energy, depending on quantum efficiency, usually at a longer wavelength. Particularly, cyanine dyes are fluorescent organic molecules characterized by a delocalized electron system that spans over a polymethine bridge and is confined between two nitrogen atoms. Some of them, having favourable optical properties, low toxicity and good solubility in aqueous media, can be used as contrast agents for biomedical imaging. Cyanine dyes with red to near-infrared emission (650-900 nm) are particularly useful for biomedical imaging applications due to the higher penetration depth compared to dyes with fluorescence emission in the visible spectrum. Among the near-infrared dyes used for biomedical imaging, Indocyanine green (ICG) is the only medicinal product currently approved for human use. ICG is routinely used to assess tissue perfusion and for angiographic applications due to the strong binding to plasma protein (blood pool effect) and rapid clearance of the unbound fraction by the liver (Cherrick et al., J Clin Invest 1960;39(4): 592-600). Furthermore, ICG is also tested as investigational medicinal product for tumor imaging during diagnostic and interventional (fluorescence-guided surgery) procedures (Tummers Q. et al., PlosOne 2015;10(6): e0129766).

Further contrast agents for fluorescence imaging are under development which exploit the use of a dye conjugated to a carrier moiety (i.e., biomolecule), targeting an overexpressed tumor epitope, to improve sensitivity and specificity of detection (Achilefu S. et al, J Med Chem 2002; 45, 2003-2015). For instance, ICG has been conjugated to a tumor-targeting moiety and is currently under evaluation in clinical trials for intraoperative tumor detection (Fidel J. et al., Cancer Res. 2015;15; 75(20): 4283-4291). ICG is an example of a near-infrared cyanine dye with inherent fluorescence emission. ICG emits fluorescence upon excitation already after administration in the bloodstream, and the fluorescence intensity detected is function of the accumulation rate of the dye in a given tissue. This results in fluorescence emission from the tissue of interest, i.e., a tissue affected by a particular physiological or pathological feature of diagnostic interest.

However, a caveat of this approach is the relatively high fluorescence intensity originated from a non-target (background) tissue in which the dye may distribute and accumulate due to non-specific retention. High fluorescence intensity arising from background regions may negatively affect the quality of a diagnostic optical imaging procedure by reducing the image contrast, i.e., the ratio between the signal in the target tissue and the signal in the background region, leading to low sensitivity of detection and false negatives.

To overcome the limitation of the inherent fluorescence emission, new classes of cyanine dyes which display red to near-infrared (650-900 nm) fluorescence emission dependent on pH are being developed. In particular, only at acidic pH (e.g., < 6.2-6.8) these dyes are fully activated and fluorescent (ON), while displaying only minimal fluorescence emission (OFF) at physiological pH (7.2-7.4). This feature is of particular relevance to perform diagnostic optical imaging of pathological tissues, for instance tumor tissues, as it is known that cancer tissues have lower pH (6.2-6.8) in the extracellular space compared to healthy tissues and blood (7.2-7.4), due to the high glycolytic metabolism (known as the "Warburg effect") and the consequent extrusion of a high concentration of protons through membrane pumps (Damaghi M. et al., Front Physiol. 2013; 4:370). Furthermore, the activation of these dyes, especially when the dye is conjugated to a moiety that binds epitopes expressed on the surface of the tumor cells, can occur after internalization in the cell and trafficking within acidic organelles such as endosomes (pH 6.5-5.5) and lysosomes (pH 5.5-4.5).

This specific activation of the dye triggered by acidic pH could increase the tumor-to-background ratio and improve the tumor visualization, since only in the tumor microenvironment or only after internalization into tumoral cells driven by the overexpression of the specific receptor, the probe becomes fluorescent and the signal could be detected. Ideally, in the blood, where pH is 7.4, and in the healthy tissue, where the cellular internalization mediated by the same receptor is much lower, the fluorescence of the probe is almost switched off, generating a very low background signal.

For the above reasons, such "activatable" (on-off) dyes may allow a better detection of the pathological regions and improve the quality of biomedical optical imaging procedures.

Some examples of pH sensitive cyanines are reported in the prior art.

For instance, WO00/75237 describes some examples of pH sensitive cyanine dyes and discloses new compounds having linear threemethine (Cy3), pentamethine (Cy5) or heptamethine (Cy7) scaffolds, non-alkylated or monoalkylated on the indolenine.

Further disclosures regarding monoalkylated cyanine dyes with red to near-infrared (650-900 nm) emission characterized by pH responsiveness for use as optical diagnostic agents are represented for example by paper Wycisk V. et al., ACS Omega 2016, 1, 5, 808-817, focused on a process for preparing water-soluble pH-sensitive cyanine dyes, and conjugates thereof, with pKa values in the region of physiological pH; and Donskyi I.S. et al, Langmuir 2017, 33, 26, 6595-6600, disclosing the conjugation of an pH-sensitive indocarbocyanine (IDCC) dye with fullerene-polyglycerol amphiphiles (FPAs) and the investigation of their physicochemical properties.

Moreover, Xue F. et al, Chem. Commun., 2017, 53, 6424-6427 reports a pH-responsive photothermal ablation agent (pH-PTT) based on cyanine dyes and nanoparticles formed by this agent assembled with bovin serum albumin.

CN105693590 A relates to a class of near-infrared laser photothermal therapeutic reagents, referred to as PTT-pH, which are able to specifically recognize tumor cells under pH control.

Briggs M. et al., Chem. Commun. 2000, 2323-2324 reports an example of a pentamethine cyanine dye sensitive to proton concentration over a pH range of 6-9 which displayed a pKa of 7.5.

WO2004/039894 relates to the preparation of different cyanine dyes, with functional groups at different sites in the indole bases. In particular, it describes pH-dependent Cy5 dyes including the commercial dye CypHer5E (GE Healthcare) and other derivatives thereof. The use of the monoalkylated pentamethine dye CypHer5E, having a pKa approximately of 7.3, to label particles and assess cell internalization is also disclosed in Beletskii A. et al., BioTechniques 2005, 39:894-897. The chemical structures of the compound disclosed in Briggs et al. (left) and of CypHer5E (right) are reported below:

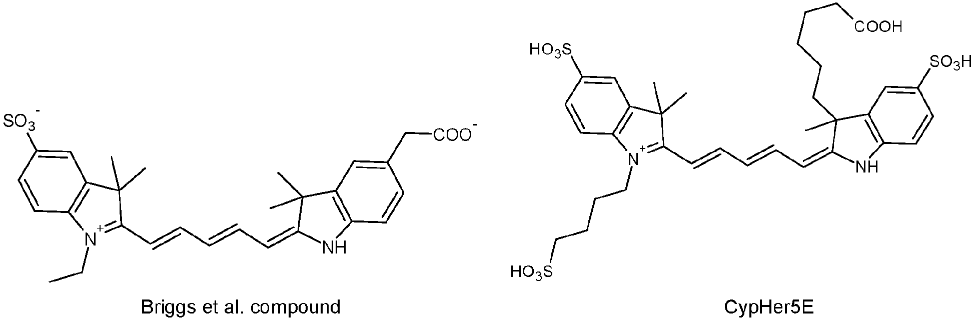

Lee H. et al., Bioconjug Chem. 2011, 22(4): 777-784 and Gilson R. et al., Mol. Pharmaceutics 2015, 12: 4237-4246 disclose non-alkylated cyclic hexenyl Cy7 pH-responsive dyes and their applications in vitro and in vivo. For instance, the first reference reports the following chloro- and benzoic acid-derivatives, having respectively a pKa of 4.7 and 5.2:

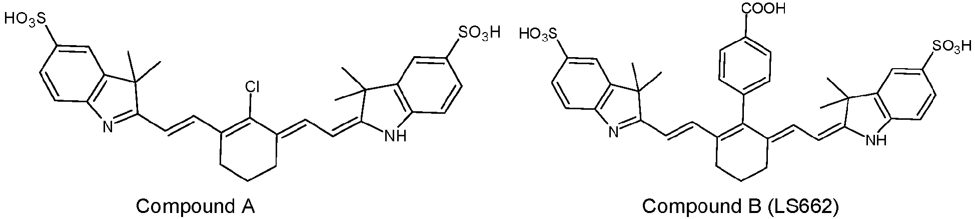

The pKa value of the compounds is of pivotal relevance since when the pH of the medium equals the pKa value (pH = pKa), only half of the dye molecules is protonated and fluorescent, whereas the other half is deprotonated and quenched. Therefore, a pH-sensitive dye with pKa >7.0 will already display high fluorescence emission (>50%) at physiological pH (7.2-7.4), which may result in high background fluorescence. The compound disclosed in "Briggs et al" paper and CypHer5E are examples of such class of pH-sensitive cyanine dyes displaying suboptimal fluorescence emission properties (i.e., high emission at physiological pH), which may result in high background fluorescence. On the other side, a pH-sensitive dye with pKa <5.5 will show high fluorescence emission (>50%) only at highly acidic pH values. For example, the above described compounds A and B would achieve 95% protonation only at pH of 3.7 and 4.2, respectively, a condition of extreme acidosis not likely to be found in tissues and cells also in pathological states. For this reason it would be preferable to develop pH sensitive dyes characterized by having a pKa in the range of 5.5-7.0, so that the fluorescence emission is minimal at physiological pH but high at mildly acidic pH.

Other drawbacks associated with the known dyes reported in Lee H. et al., Bioconjug Chem. 2011, 22(4): 777-784 and Gilson R. et al., Mol. Pharmaceutics 2015, 12: 4237-4246 are represented by a certain instability (for instance it is mentioned in the above papers that it is preferable to use freshly prepared solutions of dyes for each study) and by the fact that in several cases it is difficult to derivatize these dyes with functional groups useful for a possible conjugation to biological moieties (i.e. molecular vectors). For instance, the absence of N-alkylated groups strongly limits the possible conjugation only to one coupling site on the benzoic acid, a functional group which is not trivial to incorporate in the cyanine scaffold (Gilson R. et al., Mol. Pharmaceutics 2015, 12: 4237-4246).

Therefore, despite several efforts to find suitable imaging agents, there is still the need to find improved dyes endowed with optimal fluorescence efficiency, as well as optimal physicochemical and biological properties, and designed for optical imaging of living organisms. Dyes endowed with pKa features allowing a suitable activation and red to near-infrared (650-900 nm) fluorescence emission only in the acidic pH conditions of the body (e.g. in a tumor environment) would be preferable for applications in living organisms.

This need is paramount particularly when the dye is conjugated to a biomolecule that specifically binds a molecular epitope or a pathologic tissue (e.g. a tumor). The present invention addresses these and other needs.

### SUMMARY OF THE INVENTION

The technical field of the invention is biomedical optical imaging. In particular, the invention relates to new monoalkylated cyanine dyes with red to near-infrared (650-900 nm) emission, whose fluorescence is dependent on the pH of the medium in which they are solubilized, and to conjugates with biological ligands thereof. Furthermore, the invention relates to the use of these compounds as diagnostic agents, to the methods for their preparation and to the compositions comprising them. Generally, object of the present invention is to provide new pH responsive monoalkylated cyanine dyes, or their corresponding conjugates to binding moieties, useful as contrast medium for optical imaging and aimed at solving the above mentioned issues.

In particular, the new monoalkylated cyanine derivatives described herein have minimal fluorescence at physiological pH and are activated by mildly acidic pH, generating a fluorescent signal only in biological districts characterized by greater acidity respect to the healthy tissue or blood, thus dramatically reducing the background signal.

Moreover, the monoalkylated cyanine dyes of the invention, and conjugates thereof, are surprisingly characterized by pKa values in the range of 5.5-7.0, which are more suitable for biomedical imaging applications, and endowed with a very good responsiveness towards small changes in pH, a better stability with respect to the non-alkylated cyanine dyes of the prior art.

The new cyanine dyes can be conveniently conjugated to suitable targeting moieties through suitable functional groups acting as binding sites, thus providing very specific and sensitive contrast agents for molecular imaging.

A further aspect of the invention relates to such dyes as diagnostic agents, in particular for use in optical imaging of a human or animal organ or tissue, for use in a method of optical imaging, wherein the imaging is a tomographic imaging of organs, monitoring of organ functions including angiography, tissue perfusion imaging, urinary tract imaging, bile duct imaging, nerve imaging, intraoperative cancer identification, fluorescence-guided surgery, fluorescence endoscopy, fluorescence laparoscopy, robotic surgery, open field surgery, laser guided surgery, photodynamic therapy, fluorescence lifetime imaging, or a photoacoustic or sonofluorescence method.

Moreover the invention relates to a manufacturing process for the preparation of the provided dyes, the corresponding conjugates and/or the pharmaceutically acceptable salts thereof, and to their use in the preparation of a diagnostic agent.

According to a further aspect, the invention relates to a pharmaceutically acceptable composition comprising at least one dye or dye-conjugate compound of the invention, or a pharmaceutically acceptable salt thereof, in a mixture with one or more physiologically acceptable carriers or excipients. Said compositions are useful in particular as optical imaging agents to provide useful imaging of human or animal organs or tissues.

In another aspect, the present invention refers to a method for the optical imaging of a body organ, tissue or region by use of an optical imaging technique that comprises the use of an effective dose of a compound of the invention.

### DESCRIPTION OF THE DRAWINGS

The features of the invention can be better understood with reference to the following detailed description and the accompanying figures, wherein:

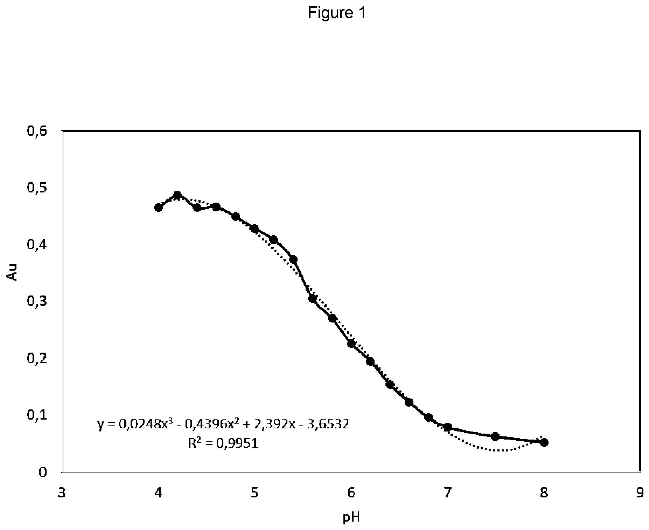

Figure 1 shows the values of maximum absorbance of a compound representative of the invention (Compound 5) at 780 nm plotted in a graph versus the pH of each buffered phosphate solution prepared from pH 4.0 to pH 8.0, in order to calculate the pK from the inflection point.

### DETAILED DESCRIPTION OF THE INVENTION

Accordingly, it is a first object of the present invention the provision of a compound of formula (I), or a pharmaceutically acceptable salt thereof, 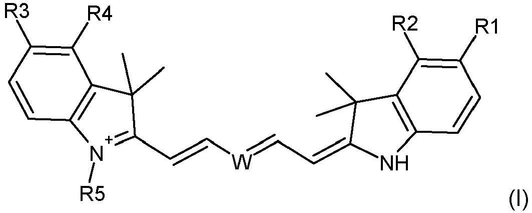 wherein
W is a group 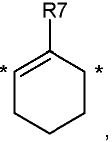 where
   **R7** is chlorine or phenyl optionally substituted by a group selected from -SO₃H, -COOH, - CONH-Y, -alkyl-COOH and -alkyl-CONH-Y, where
      Y is a bivalent alkyl substituted by -SO₃H or at least two hydroxyl groups, and
   * represents a bonding position;
**R1** and **R3** are independently selected from the group consisting of hydrogen, -SO₃H, -COOH and -CONH-Y; and **R2** and **R4** are hydrogen;
**R5** is an alkyl optionally substituted by a group selected from -SO₃H, -COOH and -CONH₂.

Another object of the present invention relates to a corresponding conjugated dye represented by a compound of formula (II), or a pharmaceutically acceptable salt thereof, 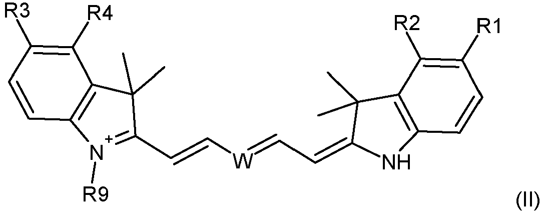 wherein
W is a group 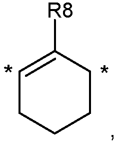 where
   **R8** is chlorine or phenyl optionally substituted by a group selected from -SO₃H, -COOH, - CONH-Y, -alkyl-COOH, -alkyl-CONH-Y and -R10, where
      **Y** is a bivalent alkyl substituted by -SO₃H or at least two hydroxyl groups,
      **R10** is a bivalent alkyl substituted by a group -CONH-(S)ₘ-T, where
         **S** is a spacer selected from -(CH₂)ₚCOO-, -(CH₂CH₂O)ₚCH₂CH₂COO- and - (CH₂CH₂O)ₚCH₂CH₂NH-, wherein p is an integer between 0 and 20;
         **T** is a targeting moiety selected from the group consisting of a small molecule, a protein, a peptide, a peptidomimetic, an enzyme substrate, an antibody or fragment thereof and an aptamer; and
         **m** is an integer equal to 0 or 1; and
   * represents a bonding position;
**R1** and **R3** are independently selected from the group consisting of hydrogen, -SO₃H, -COOH and -CONH-Y; and **R2** and **R4** are hydrogen ;
**R9** is an alkyl optionally substituted by a group selected from -SO₃H, -COOH, -CONH₂, and -CONH-(S)ₘ-T, wherein S, T and m are defined above;
and wherein at least one group -CONH-(S)ₘ-T is present in **R9** or **R10.**

The present invention also relates to methods for preparing the compounds of formula (I) or (II) by means of synthetic transformations steps.

The invention also comprises compounds of formula (I) or (II) defined above for use as fluorescent probes for biomedical optical imaging applications.

In particular the invention comprises compounds of formula (I) or (II) as defined above being characterized by pKa values in the range of 5.5-7.0.

### Definitions

In the present description, and unless otherwise provided, the following terms and phrases as used herein are intended to have the following meanings.

The term "alkyl" refers to an aliphatic hydrocarbon radical group, which may be a straight or branched-chain, having from 1 to 8 carbon atoms in the chain. For instance, "C₄ alkyl" comprises within its meaning a linear or branched chain comprising 4 carbon atoms. Similarly, "C₁-C₂₀ alkyl" is an alkyl comprising from 1 to 20 carbon atoms. Preferably and unless otherwise specified, the term "alkyl" refers to a C₁-C₆ alkyl. Representative and preferred alkyl groups include methyl, ethyl, n-propyl, iso-propyl, n-butyl, tert-butyl, pentyl and hexyl. Unless otherwise specified, the straight or branched alkyl is a monovalent radical group, otherwise it may be a multivalent radical group, wherein two or more hydrogen atoms are removed from the above hydrocarbon radical group and substituted, e.g. methylene, ethylene, iso-propylene groups and the like.

The term "bivalent alkyl" refers to an alkyl group wherein two hydrogen atoms are removed from the above hydrocarbon radical group and substituted.

The term "cycloalkyl" as used therein comprises within its meaning a saturated (i.e. cycloaliphatic) carbocyclic ring; for instance, "C₃-C₇ cycloalkyl" represents a saturated carbocyclic ring comprising from 3 to 7 carbon atoms. Suitable examples include a C₅-C₇ carbocyclic ring, e.g. a cyclohexyl ring.

The term "aryl" refers to an aromatic monocyclic or multicyclic ring system of 6 to about 14 carbon atoms, preferably of 6 carbon atoms. Representative aryl groups include phenyl, naphtyl or a benzo-fused ring. The term "benzo-fused ring" refers to an aromatic monocyclic ring of 6 carbon atoms attached to another ring forming a bicyclic aromatic system.

In the present description the term "protecting group" (Pg) designates a protective group adapted for preserving the function of the group to which it is bound. Specifically, protective groups are used to preserve amino, hydroxyl or carboxyl functions. Appropriate protective groups may include, for example, benzyl, carbonyl, such as formyl, 9-fluoromethyloxycarbonyl (Fmoc), benzyloxycarbonyl (Cbz), t-butoxycarbonyl (Boc), isopropyloxycarbonyl or allyloxycarbonyl (Alloc), alkyl, e.g. tert-butyl or triphenylmethyl, sulfonyl, acetyl groups, such as trifluoroacetyl, benzyl esters, allyl, or other substituents commonly used for protection of such functions, which are well known to the person skilled in the art (see, for instance, the general reference T.W. Green and P.G.M.Wuts, Protective Groups in Organic Synthesis, Wiley, N.Y. 2007, 4th Ed., Ch. 5).

Moreover, the invention comprises also the precursors or intermediates compounds suitable for the preparation of a desired compounds of formula (I) or (II) or salts thereof. In such derivatives the functional groups, such as a carboxylic acid or carboxamide, can be protected with an appropriate protecting group (Pg) as defined above, preferably with alkyl or ester groups. If necessary, also hydroxyl groups of Y groups can be protected with an appropriate protecting group (Pg) during the preparation of the compounds of formula (I) or (II), thus forming for instance acetoxy, alkoxy or ester groups.

The expression "coupling reagent" refers to a reagent used for instance in the formation of an amide bond between a carboxyl moiety and an amino moiety. The reaction may consist of two consecutive steps: activation of the carboxyl moiety and then acylation of the amino group with the activated carboxylic acid. Non limiting examples of such coupling agents are selected from the group consisting of: carbodiimides, such as N,N'-diisopropylcarbodiimide (DIC), N,N'-dicyclohexylcarbodiimide (DCC), 1-ethyl-3-(3-dimethylaminopropyl) carbodiimide (EDAC), 1-ethyl-3-(3-dimethylaminopropyl)carbodiimide (EDC) and 1-ethyl-3-(3-dimethylaminopropyl)carbodiimide (WSC); phosphonium reagents, such as (benzotriazol-1-yloxy)tris(dimethylamino)phosphonium hexafluorophosphate (BOP), (benzotriazol-1-yloxy)tripyrrolidinophosphonium hexafluorophosphate (PyBOP), 7-azabenzotriazol-1-yloxy-tripyrrolidino-phosphonium hexafluorophosphate (PyAOP), [ethyl cyano(hydroxyimino)acetato-O2]tri-1-pyrrolidinylphosphonium hexafluorophosphate (PyOxim), bromotripyrrolidinophosphonium hexafluorophosphate (PyBrOP) and 3-(diethoxyphosphoryloxy)-1,2,3-benzotriazin-4(3H)-one (DEPBT); and aminium/uronium-imonium reagents, such as N,N,N',N'-tetramethyl-O-(benzotriazol-1-yl)uronium tetrafluoroborate (TBTU), N,N,N',N'-tetramethyl-O-(1H-benzotriazol-1-yl)uronium hexafluorophosphate (HBTU), N,N,N',N'-tetramethyl-O-(7-azabenzotriazol-1-yl)uronium hexafluorophosphate (HATU), O-(1H-6-chlorobenzotriazole-1-yl)-1,1,3,3-tetramethyluronium hexafluorophosphate (HCTU), 1-[1-(cyano-2-ethoxy-2-oxoethylidene-aminooxy)-dimethylamino-morpholino]-uronium hexafluorophosphate (COMU) and fluoro-N,N,N',N'-tetramethylformamidinium hexafluorophosphate (TFFH) or other compounds well known to the person skilled in the art.

The expression "activated carboxylic acid" refers to a derivative of a carboxyl group that is more susceptible to nucleophilic attack than a free carboxyl group; suitable derivatives may include for instance acid anhydrides, thioesters, acyl halides, NHS ester and sulfo NHS esters.

Moreover, the terms "moiety" or "residue" are herewith intended to define the residual portion of a given molecule once properly attached or conjugated, either directly or through a suitable linker and/or spacer, to the rest of the molecule.

### Targeting moiety (T)

According to the invention, a targeting moiety **(T)** is a molecule that binds with particular selectivity to a biological target and facilitates the accumulation of the contrast agent in a specific tissue or part of the body. Generally, it is represented by a natural or synthetic molecule for use in biological systems. Such specific binding can be achieved through a ligand, such as for instance a small molecule, a protein, a peptide, a peptidomimetic, an enzyme substrate, an antibody or fragment thereof or an aptamer, interacting with a specific biological target expressed on the surface of the tissues or cells of interest.

Suitable biological targets for the compounds of the invention can be for instance an epithelial growth factor (EGF) receptor, such as EGFR or HER2; a vascular endothelial growth factor (VEGF) receptor, such as VEGFR1 or VEGFR2; a carbonic anhydrase (CA) enzyme, such as CAIX, CAll or CAXII; a mucin glycoprotein, such as MUC1; a glucose transporter, such as GLUT-1; a sodium-hydrogen antiporter, such as NHE1; a carcinoembryonic glycoprotein, such as the carcinoembryonic antigen (CEA); a chemokine receptor, such as the chemokine receptor type 4 (CXCR4); a cell adhesion molecule, such as ICAM, EPCAM, VCAM, E-Selectin, P-Selectin; the hepatocyte growth factor HGFR (c-met); a receptor for the transferrin; a ephrin receptor, such as EPHA2; a receptor for the folic acid, such as FR-alpha; a glycoprotein binding ialuronic acid, such as CD44; a bombesin receptor, such as BB1, BB2, BB3; a N-acetyl-L-aspartyl-L-glutamate (NAAG) peptidase, such as prostate-specific membrane antigen (PSMA); and, in particular, an integrin receptor, such as αᵥβ₃, αᵥβ₅, αᵥβ₆ or α₅β₁ integrin receptors.

For instance, integrin receptors targeting moieties are represented by linear or cyclic peptides comprising the sequence Arg-Gly-Asp (RGD). This tripeptide has high binding specificity for the receptor, being recognized as ligand by the family of the integrin receptors located in the cell membrane. In fact, it has been identified in some extracellular matrix glycoproteins, such as fibronectin or vitronectin, which exploit this RGD motif to mediate cell adhesion.

Therefore, linear and cyclic peptides and peptidomimetics containing the sequence Arg-Gly-Asp (RGD), such as for instance cRGD, cRGDfK, cRGDyK, cRGDfC, RGD-4C, RGD-2C, AH111585, NC100692, RGD-K5 (Kapp et al., Sci Rep, 2017, 7:3905), or their analogues and derivatives thereof, are well known examples of binding motif targeting cancer tissues on which cell membrane integrins are up-regulated compared to healthy tissues.

In one embodiment, the compounds of the invention can be conjugated to other small molecules, peptides, proteins or antibodies, such as for instance monoclonal antibodies already used for therapy. Small molecules containing the drug acetazolamide, such as for instance compounds 4a, 5a, 6a, 7a and 8a (Wichert et al., Nat Chem 2015, 7: 241-249), or their analogues and derivatives thereof, are examples of small moleculs targeting the enzyme CAIX. Linear and cyclic peptides and peptidomimetics, such as peptide GE11 (described in Li et al., FASEB J 2005, 19:1978-85) and/or peptide L1 (described in Williams et al., Chem Biol Drug Des 2018, 91:605-619), or their analogues and derivatives thereof, are examples of peptides targeting the epithelial growth factor receptor (EGFR). Among the proteins, derivatives of the epithelial growth factor (EGF) are examples of small protein targeting the epithelial growth factor receptor (EGFR). Among the antibodies, panitumumab and cetuximab are examples of monoclonal antibodies targeting the epithelial growth factor receptor (EGFR).

Preferably, the targeting ligands of the invention can selectively link tumor cells or tissues. In particular, they can link to tumors selected from brain cancer, breast cancer, head and neck cancer, ovarian cancer, prostate cancer, esophageal cancer, skin cancer, gastric cancer, pancreatic cancer, bladder cancer, oral cancer, lung cancer, renal cancer, uterine cancer, thyroid cancer, liver cancer, and colorectal cancer. In addition, the targeting ligands are able to link metastatic spreads of the above-mentioned cancers in tissues and organs different from the primary source. Furthermore, the targeting ligands are able to link pre-neoplastic lesions and dysplasia in different tissues and organs.

### Spacer S

According to the invention, **S** is a spacer, optionally present, that separates the targeting moiety from the dye. The presence of a spacer is particularly relevant for some embodiments where the targeting moiety and the dye risk to adversely interact with each other. Moreover, the presence of the spacer may be necessary when the dye is relatively large and may interfere with the binding of the targeting moiety to the target site.

The spacer can be either flexible (e.g., simple alkyl chains) or rigid (e.g., cycloalkyl or aryl chains) so that the dye is oriented away from the target. The spacer can also modify pharmacokinetic and metabolism of the conjugates of formula (II) used as imaging agents in a living organism.

Hydrophilic spacers may reduce the interaction with plasma proteins, reduce blood circulation time and facilitate excretion. For example, if the spacer is a polyethyleneglycol (PEG) moiety, the pharmacokinetics and blood clearance rates of the imaging agent *in vivo* may be altered. In such embodiments, the spacer can improve the clearance of the imaging agent from background tissue (i.e., muscle, blood) thus giving a better diagnostic image due to high target-to-background contrast. Moreover, the introduction of a hydrophilic spacer may shift the elimination of the contrast agent from hepatic to renal, thus reducing overall body retention.

Therefore, in one preferred embodiment, the spacer is a hydrophilic moiety comprising C₁-C₂₀ alkyl, C₃-C₇ cycloalkyl or aryl groups. Preferably, the spacer is selected from the group consisting of - (CH₂)ₚCOO-, -(CH₂CH₂O)ₚCH₂CH₂COO- and -(CH₂CH₂O)ₚCH₂CH₂NH-, wherein p is an integer between 0 and 20. Preferably p is 2, 6 or 12.

When not necessary, the spacer is preferably absent, i.e. m is 0 and S represents a direct bond.

The spacer, or alternatively the targeting moiety when the spacer is absent, can be connected to a compound of formula (I), alternatively at the R5 and/or R7 residue, to form a compound of formula (II). The linking groups of R5/R7 are reactive functional groups such as carboxylic acid residues suitable for conjugating the dye to the targeting moiety by formation of a chemical bond.

For instance, when an amine-containing targeting moiety (T) is conjugated with a compound of formula (I) wherein R5 and/or R7 is an alkyl substituted by carboxylic acid, this carboxylic acid may be optionally activated before carrying out the conjugation through conversion in a more reactive form using an activating reagent, forming for example a N-hydroxy succinimide (NHS) ester or a mixed anhydride. Then, to obtain the corresponding compound of formula (II), the amine-containing targeting moiety is treated with the resulting activated acid to form an amide linkage. Typically, this reaction is carried out in aqueous buffer, optional co-solvent with DMSO or DMF at pH 8 to 9, or in organic solvent with organic bases such as DIPEA, TEA or NMM.

Otherwise a direct conjugation using the "non-activated" carboxylic acid may be performed. Similarly, when the linking group of R5 is a carboxamido group, the procedure for attachment of the suitable targeting moiety is analogous, but no activation step of the linker is generally required and the dye and targeting moiety are treated directly.

The compounds of the above formula (I) or (II) may have one or more asymmetric carbon atoms, otherwise referred to as chiral carbon atoms, and may thus give rise to diastereomers and optical isomers. Unless otherwise provided, the present invention further includes all such possible diastereomers as well as their racemic mixtures, their substantially pure resolved enantiomers, all possible geometric isomers, and pharmaceutically acceptable salts thereof.

The present invention further relates to compounds of the above formula (I) or (II) in which the functional groups of R1, R3, R5, R7, R8, R9 and Y, e.g. the sulfonyl, carboxamido or carboxylic acid groups, may be in the form of a pharmaceutically acceptable salt.

In detail, the invention relates to a compound of formula (I) or (II) wherein W is respectively a group 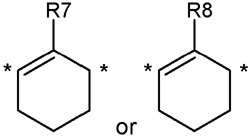 R1 and R3 are independently selected from the group consisting of hydrogen, -SO₃H, -COOH and -CONH-Y, where Y is a bivalent alkyl substituted by -SO₃H or at least two hydroxyl groups, R2 and R4 are hydrogen, and R5, R7, R8 and R9 are as defined above, otherwise represented by the following formulae (Ib) or (Ilb) respectively:

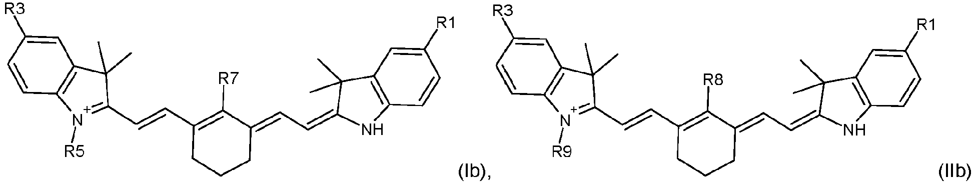

In one embodiment, the invention relates to a compound of formula (I) or (II) wherein Y is a linear or branched C₁-C₆ alkyl substituted with from two to five hydroxyl groups.

In a preferred embodiment the invention relates to a compound of formula (I) or (II) wherein Y is selected from the group consisting of

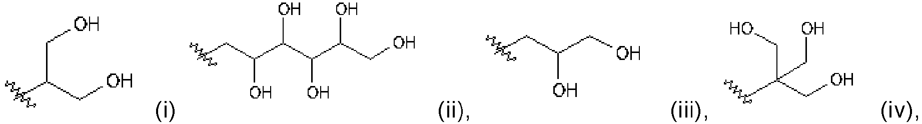

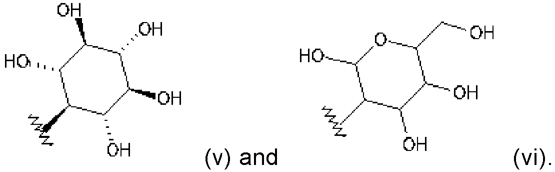

More preferably, the invention relates to a compound of formula (I) or (II) wherein Y is a group of formula (ii) as defined above.

Another embodiment of the invention relates to a compound of formula (II) wherein m is 0 and the spacer S is represented by a direct bond or m is 1 and the spacer is a hydrophilic moiety comprising C₁-C₂₀ alkyl, C₃-C₇ cycloalkyl or aryl groups. Preferably, the spacer is selected from -(CH₂)ₚCOO-, -(CH₂CH₂O)ₚCH₂CH₂COO- and -(CH₂CH₂O)ₚCH₂CH₂NH-, wherein p is an integer between 0 and 20. Preferably p is 2, 6 or 12.

According to the invention, T is a targeting moiety selected from a small molecule, a protein, a peptide, a peptidomimetic, an enzyme substrate, an antibody or any fragment thereof and an aptamer.

Preferably T is represented by a peptide, and in particular by a moiety interacting with an integrin receptor, such as αᵥβ₃, αᵥβ₅, αᵥβ₆, α₅β₁ and the like, preferably with αᵥβ₃ integrin receptor. Especially preferred are the compounds of formula (I) listed in Table la and the related conjugated compounds of formula (II) listed in Table Ib.

**Table la - Preferred compounds of formula (I)**

| **Compound** | **Structure** |
|---|---|
| Refer. Compound A *(*Lee et al., Bioconjug Chem 2011; 22(4): 777-784) | 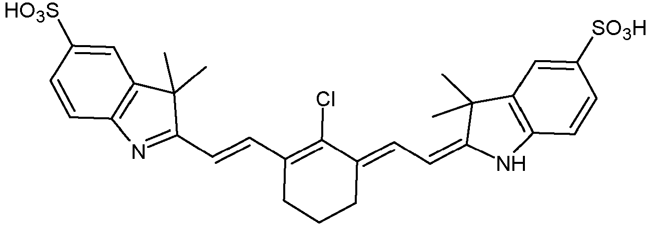 |
| Compound 1 | 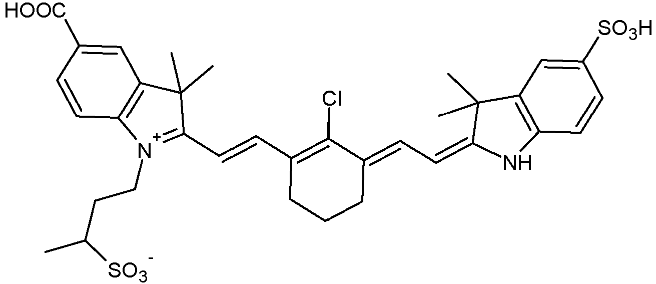 |
| Compound 2 | 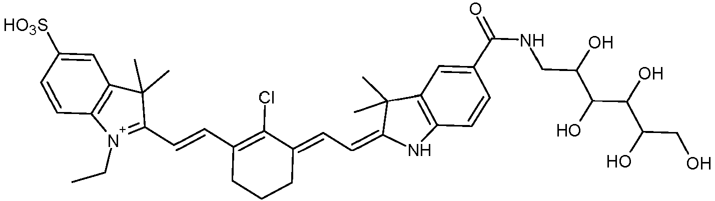 |
| Compound 3 | 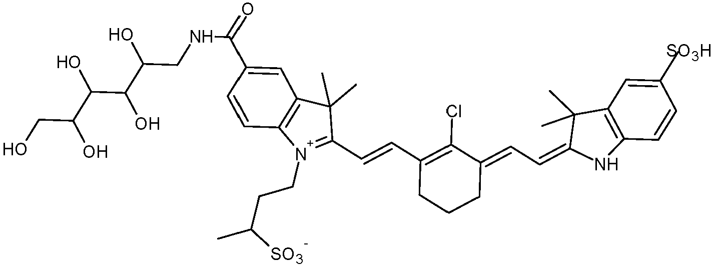 |
| Compound 4 | 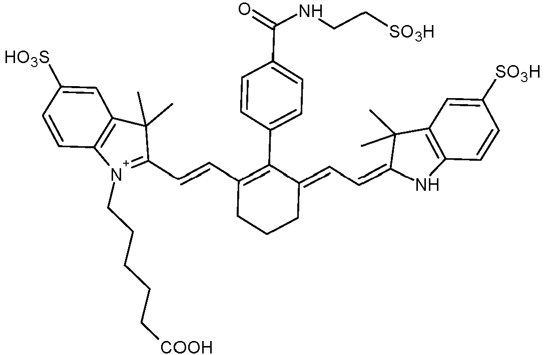 |
| Compound 5 | 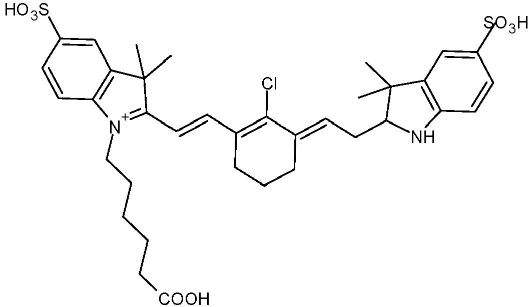 |
| Compound 6 | 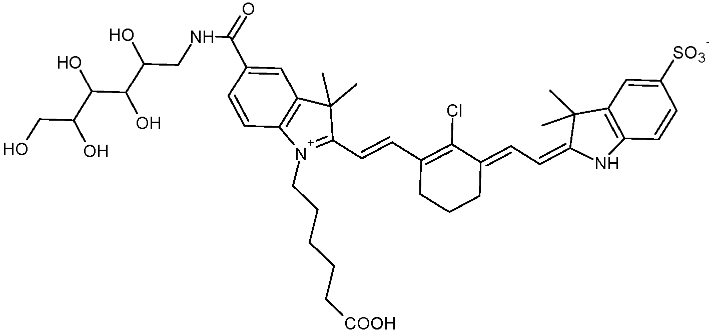 |
| Compound 7 | 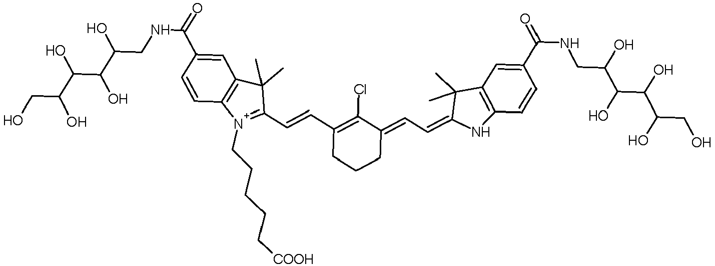 |

**Table Ib - Preferred compounds of formula (II)**

| **Compound** | **Structure** |
|---|---|
| Compound 10 | 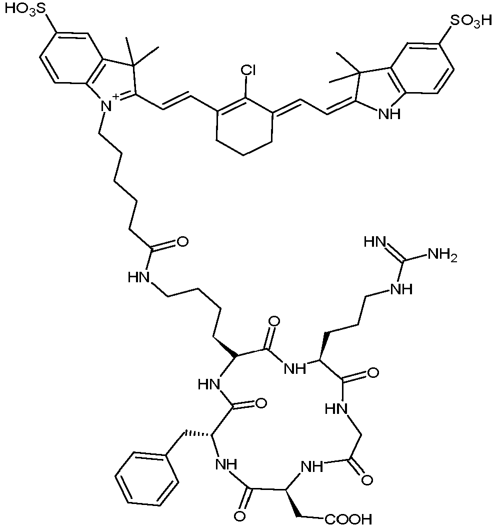 |
| Compound 11 | 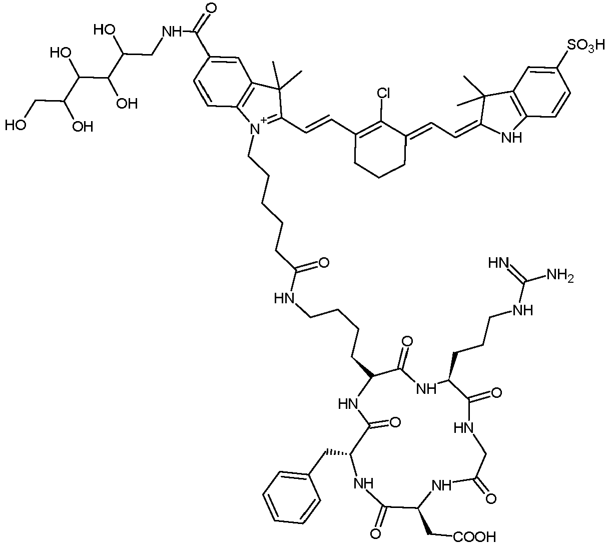 |
| Compound 12 | 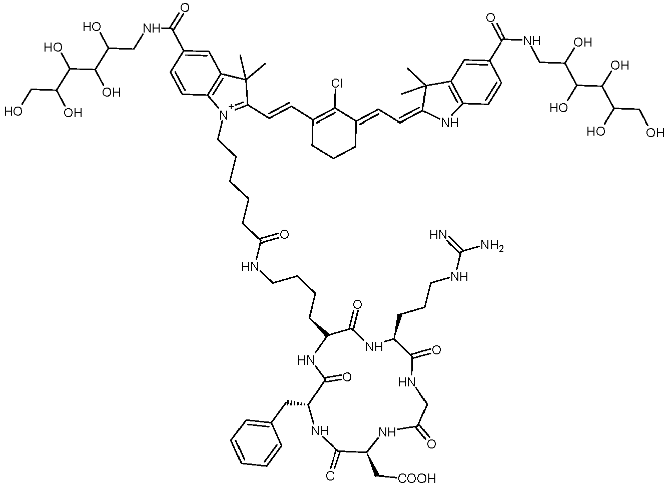 |

The present invention is also directed to methods for synthesizing the compounds of formula (I) or (II), prepared as illustrated in the following of the description, which are dyes with red to near-infrared (650-900 nm) emission optionally conjugated to a targeting moiety through a linking group.

Accordingly, the invention provides the compounds of formula (I) or (II) as defined above for use as optical imaging agents for diagnostic biomedical applications in mammals (humans and animals). Preferably the imaged mammal subject is a human.

In a preferred embodiment, the compounds of the invention are for use as imaging agents in the detection of normal (healthy) tissues or abnormal (pathologic) tissues, in particular a tumor.

Preferably, the compounds of formula (I) or (II) as defined above are for use in the detection of normal (healthy) tissues by means of imaging techniques comprising for instance angiography, perfusion imaging, bile duct imaging and nerve imaging.

In a further preferred embodiment, the invention provides for compounds of formula (I) or (II) as defined above for use in the detection of abnormal (pathologic) tissue such as for instance a primary tumor lesion, local or distant metastases, or a pre-neoplastic lesion, in particular dysplasia and hyperplasia. In particular, the compounds of formula (II) as defined above are preferably for use in the detection and demarcation of a tumor margin in guided surgery of an individual patient. A preferred use is wherein said tumor is a tumor showing an overexpression of a biological epitope, for instance selected from a receptor, an enzyme, a glycoprotein, a lipid raft, a transmembrane protein located on the cell surface and a soluble factor present in serum, plasma or the interstitial space. Preferably, said biological epitope is an integrin receptor for Vitronectin, Fibrogen and/or for the transforming growth factor-β (TGF-β).

The invention also provides a compound of formula (I) or (II) for use as fluorescent probe as defined above, wherein the detection and demarcation of the tumor is carried out under NIR radiation. Preferably, such detection and demarcation of tumor is carried out before, during or after a surgical procedure to remove such tumor tissue. A fluorescence-guided surgery procedure is an example of such use.

Additionally, the invention provides compounds of formula (I) or (II) as defined above for use in the detection of an inflamed tissue, a fibrotic tissue, an ischemic tissue, or a tissue with abnormal metabolic rate.

The invention also provides compounds of formula (I) or (II) as defined above for use in a method of imaging tissues and cells comprising the steps of:
i) contacting the cells or tissues with a compound of formula (I) or (II);
ii) irradiating the tissues or cells at a wavelength absorbed by the imaging agent;
iii) detecting the near-infrared emission using a fluorescence camera.

Preferably, said contacting the cells or tissues with the imaging agents of formula (I) or (II) is accomplished by topical or local application (e.g., by spraying, soaking or applying an ointment, foam or cream) or by systemic application (enteral or parenteral administration). The invention further relates to a pharmaceutical diagnostic composition comprising a compound of formula (I) or a conjugate of formula (II) as defined above, and at least one pharmaceutically acceptable carrier or excipient.

In particular, the invention relates to a pharmaceutical composition comprising a dye of formula (I), or a salt thereof, and one or more pharmaceutically acceptable adjuvants, excipients or diluents. Alternatively, the invention relates to a pharmaceutical composition comprising a conjugate of formula (II) wherein R9 and/or R10 is alkyl substituted with CONH-(S)ₘ-T as defined above, or a salt thereof, and one or more pharmaceutically acceptable adjuvants, excipients or diluents.

Another aspect of this invention relates to a diagnostic kit comprising a compound of formula (I) or (II) as defined above. In addition, the kit can contain additional adjuvants for implementing the optical imaging. These adjuvants are, for example, suitable buffers, vessels, detection reagents or directions for use. The kit preferably contains all materials for an intravenous administration of the compounds of the invention.

The compounds of the invention may be administered either systemically or locally to the organ or tissue to be imaged, prior to the imaging procedure. For instance, the compounds can be administered intravenously. In another embodiment they may be administered parenterally or enterally.

The compositions are administered in doses effective to achieve the desired optical image of a tumor, tissue or organ, which can vary widely, depending on the compound used, the tissue subjected to the imaging procedure, the imaging equipment being used and the like.

The exact concentration of the imaging agents is dependent upon the experimental conditions and the desired results, but typically may range between 1 nM to 0.1 mM. The optimal concentration is determined by systematic variation until satisfactory results with minimal background fluorescence are obtained.

Once administered, the imaging agents of the invention are exposed to a light, or other form of energy, which can pass through a tissue layer. Preferably the radiation wavelength or waveband matches the excitation wavelength or waveband of the photosensitizing agent and has low absorption by the non-target cells and the rest of the subject, including blood proteins.

Tipically, the optical signal is detectable either by observation or instrumentally and its response is related to the fluorescence or light intensity, distribution and lifetime.

### Description of the syntheses

The preparation of the compounds of formula (I) or (II), as such or in the form of physiologically acceptable salts, represents a further object of the invention. The cyanine dyes and dye-conjugates of the invention can be prepared for instance according to the methods described in the following sections and in the experimental part.

A general teaching about the preparation of cyanine dyes can be found in Mujumdar R.B. et al., Bioconjugate Chem. 1993, 4(2): 105-111, which relates to the synthesis and labeling of sulfoindocyanine dyes. However, the cyanines of the present invention are characterized by a specific functionalization pattern not present in the compounds of the art, for which the set up of a proper synthetic approach was required. In fact, unlikely other known cyanines, the compounds of the invention can bear even three functional moieties (carboxylic acid or amido groups) to be derivatized in different ways, so that the use of protecting groups is necessary in most cases to direct the reactions on the desired functional group.

It is known that difficulties can arise when manipulating the cyanines at the strong pH and temperature conditions necessary for the removal of some protecting groups, since the stability of the polymethine scaffold can be compromised in some cases, with severe degradation of the dyes. Moreover, further obstacles can be encountered due to a possible hydrolysis and degradation of the amide groups -CONH-Y when deprotecting an ester group (typically, amide derivatives can be hydrolyzed in concentrated alkaline medium, see for instance Yamana et al, Chem. Pharm. Bull., 1972, 20(5), 881-891).

In one preferred embodiment, the protective group for the moiety R5 or R9 is an ester group. More preferably, an ethyl ester group can be advantageously used.

### Preparation of cyanine dyes of formula (I)

According to the invention, compounds of formula (I) can be prepared through a sequence of synthetic steps as reported in the following Schemes. Different synthetic routes can be selected based on the substituents and specific scaffold of the cyanine dyes.

For instance, in case of cyanines of formula (Ib) wherein W is group -cyclohexenyl-R7, where R7 is chlorine, R1 and R3 are independently selected from the group consisting of hydrogen, -SO₃H, -COOH and -CONH-Y, where Y is a bivalent alkyl substituted by -SO₃H or at least two hydroxyl groups, the procedure reported in the following general Scheme 1 can be applied.

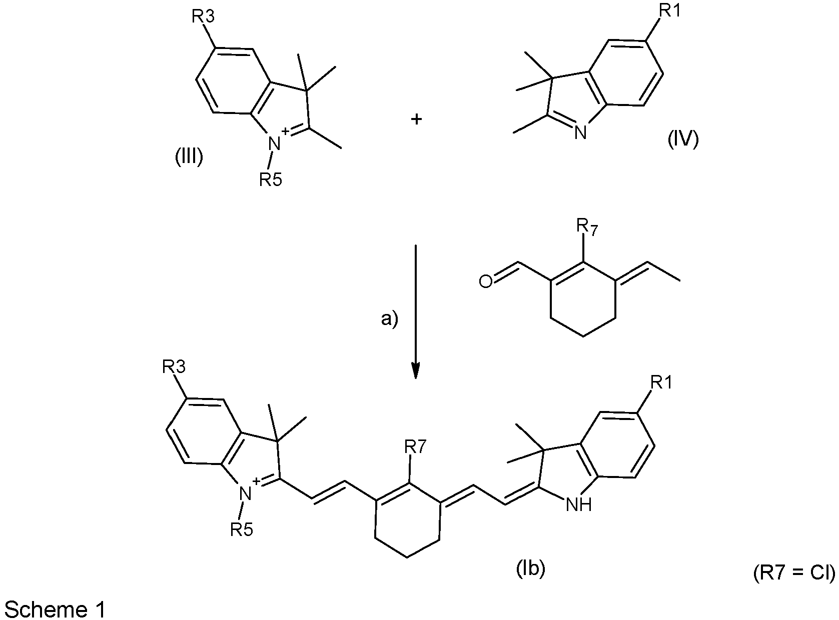

Accordingly, a process of the present invention comprises the following step:
a) reacting suitable amounts of the 5-substituted-2,3,3-trimethylindolenine intermediates of formula (III) and (IV) with 1-formyl-3-(hydroxymethylene)-1-cyclohexene or 2-chloro-1-formyl-3-(hydroxymethylene)-1-cyclohexene, to obtain the cyanine of formula (Ib), wherein R7 is chlorine atom.

According to step a) the reaction can be performed using the Vilsmeier reagent in the bis anilido form or in the bis aldehyde form (as reported in Scheme 1). The reaction can be carried out in several solvents such as for example ethanol, methanol, acetic anhydride or acetic acid or a mixture, with or without the addition of different bases, such as trimethylamine, pyridine, sodium acetate, potassium acetate etc., stirring the mixture at different temperatures ranging from 45°C to 120°C for several hours (typically 2-24 hours).

When R1 and/or R3 are independently a group -COOH, the compound of formula (Ib) can be further derivatized to obtain another compound of formula (Ib) wherein R1 and/or R3 are a group -CONHY. According to this step, the conversion of a carboxylic acid of formula (Ib) into the corresponding carboxamide can be accomplished in a variety of ways and experimental conditions, which are widely known in the art for preparation of carboxamides. As an example, the carboxylic acid can be first converted in a suitable activated ester and then reacted with an ammonium salt, such as NH₄Cl, preferably in the presence of a coupling agent, such as HBTU.

When derivatives (III) and (IV), wherein R1 and/or R3 are a group -COOH, are derivatized with polyhydroxylated amines, the reaction can be carried out by activation of the carboxylate group with a coupling agent, for instance selected from HATU, TBTU, HBTU, PyBOP, DCC, DSC and DCC-NHS, and an organic base, such as TEA, DIPEA, NMM or pyridine, in a solvent such as dimethylformamide, dimethylacetamide, dimethylsulfoxide, acetonitrile etc, at room temperature for a suitable time ranging from 30 minutes to several hours. This derivatization of the carboxylic acid can be perfomed on the alkylated indolenine or on the indole, prior quaternarization. In this case, it is important to protect the hydroxyl groups of the polyhydroxilated amines with a suitable protecting group such as acetyl, before the alkylation with sultone or bromo-hexanoic acid. This alkylation can be performed neat or in a high boiling solvent, such as butyrronitrile, sulfolane, 1,2-dichlorobenzene, dimethylacetamide, dimethylformamide or dimethylsulfoxide, stirring the solution at high temperature, for instance between 90°C and 180°C, for several hours, typically from 12 hours to 5 days.

For the embodiments relating to a cyanine of formula (Ib) wherein W is group -cyclohexenyl-R7, where R7 is phenyl optionally substituted with a group -SO₃H, -COOH, -CONH-Y, -alkyl-COOH or -alkyl-CONH-Y, where Y is a bivalent alkyl substituted by -SO₃H or at least two hydroxyl groups, and R1 and R3 are independently selected from hydrogen, -SO₃H, -COOH and -CONH-Y, the procedure reported in the following general Scheme 2 can be applied:

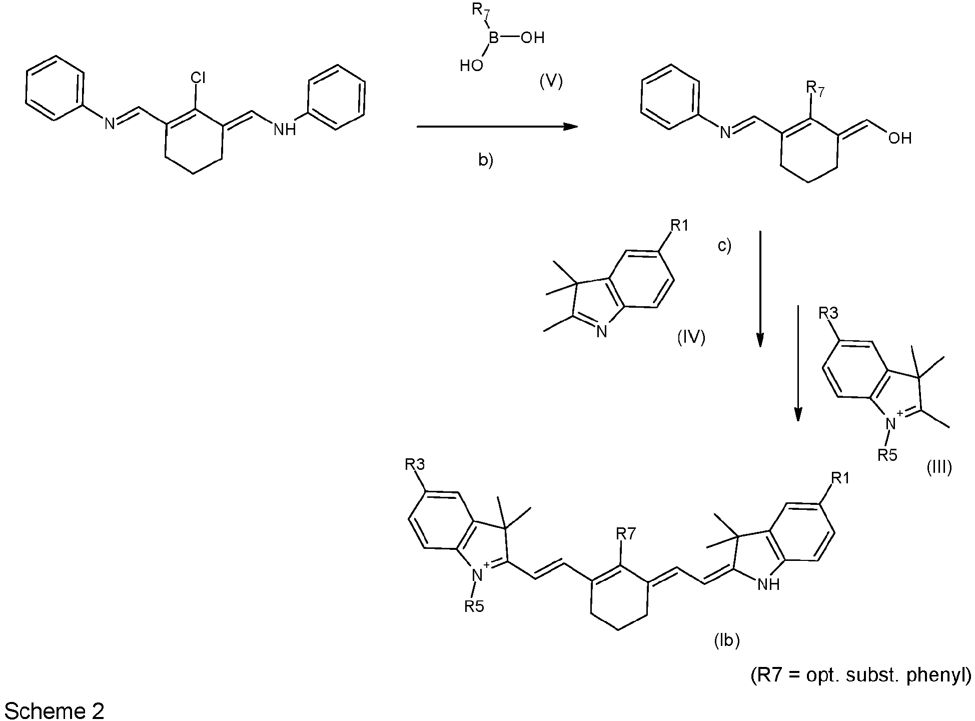

Accordingly, a process of the present invention comprises the following step:
b) reacting N-[(3-(anilinomethylene)-2-chloro-1-cyclohexen-1-yl)methylene]aniline with a suitable phenylboronic acid of formula (V) to obtain the corresponding intermediate of formula (VI); and
c) adding to the mixture comprising intermediate (VI) first the intermediate (IV) and then the intermediate (III) to obtain a compound of formula (Ib) as defined above.

According to step b), the reaction can be carried out in several solvents such as ethanol, methanol, water or a mixture thereof, in the presence of a Palladium catalyst such as palladium acetate or tetrakis(triphenylphosphine)palladium and an inorganic base such as sodium carbonate or potassium carbonate, stirring the mixture at different temperatures ranging from 70°C to 100°C for several hours (typically 2 hours).

According to step c), the reaction can be performed in several solvents such as ethanol or methanol stepwise, adding first the so-modified Vilsmeier reagent prepared in b) and the non-alkylated indolenine (IV) in the presence or absence of small amounts of acetic acid and stirring the solution at 50-80 °C for several hours (typically 4 hours). Then, the alkylated indolenine (III) is added, dissolved in an organic solvent such as ethanol or methanol, followed optionally by a base such as pyridine, triethyl amine, sodium or potassium acetate. The dark red solution can be stirred at 50-80°C for several hours, typically from 4 to 96 hours.

In all those embodiments, the functional groups of R5 and/or R1/R3 can be optionally protected during the syntheses with one or more suitable protecting groups, which need to be removed in a subsequent step. The products obtained from steps a)-d) can be deprotected according to the known procedures, described for instance in T.W. Green and P.G.M.Wuts, Protective Groups in Organic Synthesis, Wiley, N.Y. 2007, 4th Ed., Ch. 5.

### Preparation of conjugate compounds of formula (II)

The cyanine derivatives of formula (I), or salts thereof, can be conjugated with a suitable targeting moiety, optionally with the insertion of a spacer, to obtain the corresponding compounds of formula (II).The conjugation can be accomplished following different procedures known in the art, such as for instance via direct coupling of a carboxylic acid group of the compounds with a nucleophilic residue of the targeting moiety, or optionally with the spacer, or by previous activation, wherein the carboxylic acid group is transformed in a more reactive group, e.g. an ester such as NHS, before the coupling.

If a compound of the formula (I) or (II) prepared according to the processes described above is obtained as mixture of isomers, their separation using conventional techniques into the single corresponding isomer of the formula (I) or (II) is within the scope of the present invention.

The final compounds may be isolated and purified using conventional procedures, for example chromatography and/or crystallization and salt formation.

A compound of formula (I) or (II) as defined above can be converted into a pharmaceutically acceptable salt. The compounds of formula (I) or (II) as defined above, or the pharmaceutically acceptable salt thereof, can be subsequently formulated with a pharmaceutically acceptable carrier or diluent to provide a pharmaceutical composition.

### EXPERIMENTAL PART

The invention and its particular embodiments described in the following part are only exemplary and not to be regarded as a limitation of the present invention: they show how the present invention can be carried out and are meant to be illustrative without limiting the scope of the invention.

### Materials and Equipment

All commercially available reagents used in the synthesis were obtained from Sigma Aldrich and TCI and they were used without further purification. c(RGDfK) was purchased from Apex Bio or Bachem. All the reactions were followed by HPLC (Agilent mod. 1100/1200) and HPLC-MS (Agilent mod. 1260, Quadrupole LC/MSD Mod. 6120) equipped with an absorption detector set at different wavelengths (Column: YMC-Triart Phenyl, 250 x 4.6 mm / S-5 µm / 12 nm). Purifications were performed by flash chromatography on an automated purification system (CombiFlash^{®} Rf+), using pre-packed silica C18 cartridges (Biotage^{®} or Phenomenex) or by preparative HPLC on preparative YMC-Triart Phenyl column. Absorbance, excitation and emission values were assessed in a well plate reader to speed-up the measurements (SPARK).

### List of abbreviations

- DCC: N,N'-dicyclohexylcarbodiimide
- DIPEA: N,N-Diisopropylethylamine
- DMF: Dimethylformamide
- DMSO: Dimethyl sulfoxide
- DSC: N,N'-Disuccinimidyl carbonate
- HATU: 1-[Bis(dimethylamino)methylene]-1H-1,2,3-triazolo[4,5-b]pyridinium 3-oxid hexafluorophosphate
- HBTU: O-Benzotriazol-1-yl-N,N,N',N'-tetramethyluronium hexafluorophosphate
- HPLC: High performance liquid chromatography
- PBS: Phosphate buffered saline
- NHS: N-hydroxysuccinimide
- NMM: N-methylmorpholine
- RT: Room temperature
- PyBOP: (benzotriazol-1-yloxy)tripyrrolidinophosphonium hexafluorophosphate
- TEA: Triethylamine
- TBTU: 2-(1H-Benzotriazole-1-yl)-1,1,3,3-tetramethyluronium tetrafluoroborate
- TSTU: O-(N-Succinimidyl)-1,1,3,3-tetramethyluronium tetrafluoroborate
- µL: Microliter
- µM: Micromolar
- t_{R}: Retention time (HPLC)
- c(RGDfK): Cyclo-(Arg-Gly-Asp-D-Phe-Lys)

The abbreviations for individual amino acids residues are conventional: for example, Asp or D is aspartic acid, Gly or G is glycine, Arg or R is arginine. The amino acids herein referred to should be understood to be of the L-isomer configuration unless otherwise noted.

### Example 1: Synthesis of Compound 1

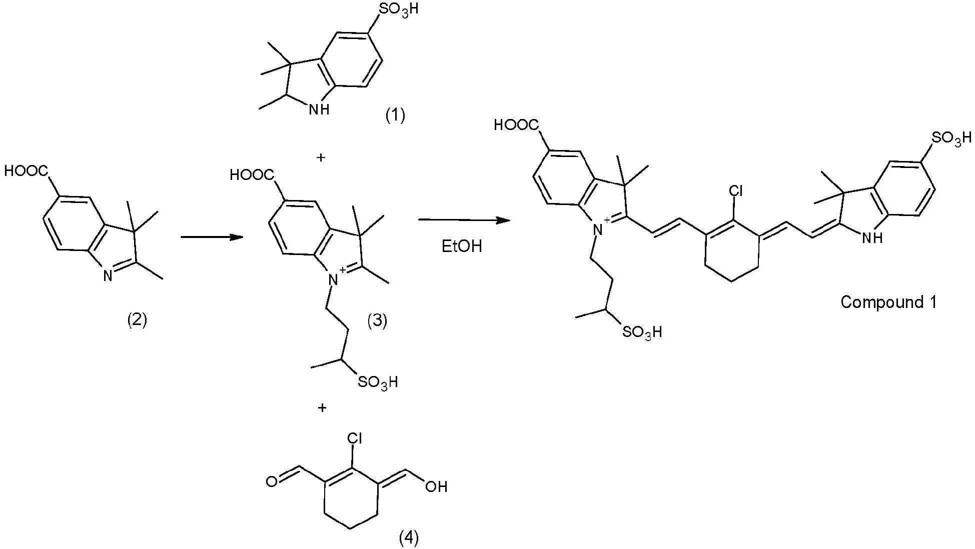

### Preparation of intermediate (2)

Glacial acetic acid (225 mL) was added to a mixture of 4-hydrazinobenzoic acid (15 g, 98.6 mmol), methyl isopropyl ketone (21.1 mL, 197.2 mmol) and sodium acetate (16.2 g, 197.2 mmol) in a round-bottom flask fitted with a condenser under magnetic stirring. The orange suspension was heated under reflux, turning into a dark brown solution. After 3 hours the reaction was completed, the solvent was concentrated under reduced pressure, the residue was dissolved in dichloromethane (300 mL) and extracted with a saturated solution of NaHCO₃ (3x100 mL). The organic phases contained most of the product; the aqueous phases were re-extracted with dichloromethane (150 mL) and all the organic phases were combined. The whole organic phases were concentrated under reduced pressure, obtaining a solid (20.43 g) that was crystallized with Ethyl Acetate/Petroleum Ether 3:1 (135 mL). The first solid was filtered and the mother liquors were concentrated and re-crystallized again with a solution of EtOAc/PE 1:1.5 (25 mL), obtaining a second solid at high purity that was combined with the previous one and dried under vacuum, obtaining 13.31 g, 66% yield.

### Preparation of intermediate (3)

Intermediate (2) (3.7 g, 18.2 mmol) was suspended in butyronitrile (12 mL) in a round-bottom flask fitted with a condenser under magnetic stirring. Then, 2,4-butanesultone (2.09 mL, 20 mmol) was added. The orange suspension was heated under reflux, turning into a dark brown solution. After 42 hours the reaction was completed: the solution was cooled to RT and 110 mL of acetone were added under magnetic stirring. After 2 hours the solid was filtered off and the solid was dried at 35°C under vacuum overnight (6.03 g, 98% yield).

HPLC purity at 270 nm: 95.1%. MS: [M + H]⁺ 341.0.

### Synthesis of Compound 1

In a round bottom flask, dried under nitrogen atmosphere, intermediate (1) (91.60 mg, 0.386 mmol, prepared as reported in US Patent Nr. 7,408,062) and intermediate (2) (100.0 mg, 0.295 mmol) were dissolved in EtOH (40 mL) at 50°C and then 2-chloro-3-(hydroxymethylene)-1-cyclohexene-1-carboxaldehyde (50.86 mg, 0.295 mmol) was added. The mixture was then heated at 50°C and stirred under nitrogen flow. The red starting mixture became purple-red after 2 minutes of reaction. After 20 hours at 50°C, the reaction was stopped. The crude mixture was dried under vacuum and dissolved in the minimum amount of water. Then, 1 mL of HCl 1M was added (pH of the mixture 1.92) and the crude was purified by flash chromatography on a pre-packed Phenomenex AQ C18 spherical silica column (40 g) with a milliQ water-acetonitrile gradient. Fractions containing the pure product were combined, distilled under vacuum, and freeze-dried, giving a dark green solid (50.55 mg, 24.0% yield). HPLC purity at 510 nm: 97.3%. MS: [M + H]⁺ 715.2.

### Example 2: Synthesis of Compound 2

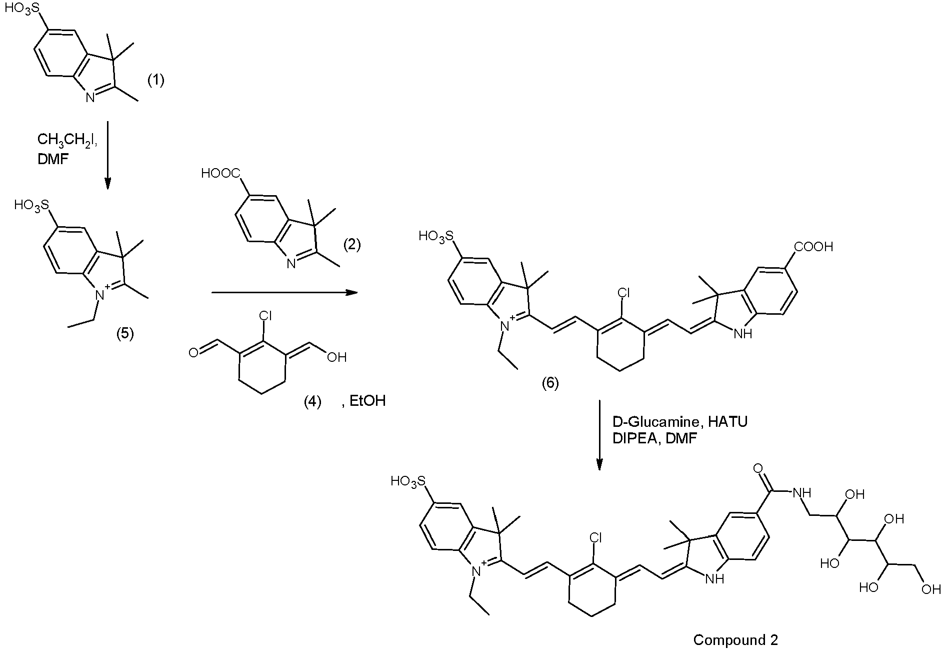

### Preparation of intermediate (5)

In a round bottom flask, dried under nitrogen atmosphere, the intermediate (1) (1.00 g, 3.83 mmol) was dissolved in dry DMF (3 mL), and iodoethane (2.02 mL, 25.1 mmol) was added at RT. The orange mixture was kept under magnetic stirring at 130°C for 5 hours. After 1 hour at 130°C the reaction mixture became pink-red. After 5 hours the crude solid was suspended in 20 mL of cold diethyl ether and the dispersion was kept under stirring for 30 minutes. The solid was filtered and washed with cold diethyl ether and further suspended in cold acetone. The dispersion was kept under stirring for 30 minutes and subsequently filtered. The solid was dissolved in 1.5 mL of MeOH and the concentrated solution was dropped into 50 mL of cold acetone. The dispersion was kept under stirring for 1 hour and subsequently filtered and the resulting solid was washed with cold acetone. Finally, the crude red solid was purified on a pre-packed C18 silica column (Biotage^{®} SNAP ULTRA, 60 g) with a 0.01% formic acid/acetonitrile gradient. Fractions containing the pure product were combined, distilled under vacuum and freeze-dried, giving a pink solid (297.4 mg, 29.0% yield). HPLC purity at 270 nm: 97.6%. MS: [M + H]⁺ 268.8.

### Preparation of intermediate (6)

In a round bottom flask, dried under nitrogen atmosphere, the intermediates (5) (232.4 mg, 0.869 mmol) and (2) (231.5 mg, 1.14 mmol) were dissolved in EtOH (65 mL) and 2-chloro-3-(hydroxymethylene)-1-cyclohexene-1-carboxaldehyde (150.1 mg, 0.869 mmol) was added at RT. The mixture was then heated at 50°C and stirred under nitrogen flow. The yellow starting mixture became red after 30 minutes of reaction. After 20 hours at 50°C, the reaction was stopped and the solvent was distilled under reduced pressure. The purification was performed by flash chromatography on pre-packed C18 silica columns (Biotage^{®} SNAP ULTRA, 60 g) with a milliQ water-acetonitrile gradient. Fractions containing the pure product were combined, distilled under vacuum and freeze-dried, giving a dark green solid (19.55 mg, 3.8% yield). HPLC purity at 520 nm: 98.9%, 20.5% at 780 nm. MS: [M + H]⁺ 607.2.

### Synthesis of Compound 2

In a round bottom flask, dried under nitrogen atmosphere, the intermediate (6) (13.5 mg, 0.022 mmol) was dissolved in dry DMF. D-Glucamine (9.64 mg, 0.053 mmol), DIPEA (15.5 µL, 0.089 mmol) and HATU (21.1 mg, 0.056 mmol) were added. The dark red solution was kept under magnetic stirring at RT for 5 hours, then cold diethyl ether (60 mL) was added. The dispersion was kept under stirring for 2 hour and subsequently stored at -20°C for 48 hours. The obtained solid was filtered and washed with cold diethyl ether, dissolved in MeOH and dried under vacuum. The obtained green solid was purified on a pre-packed C18 silica column (Biotage^{®} SNAP, 12 g) with a milliQ water-acetonitrile gradient. Fractions containing the pure product were combined, distilled under vacuum and freeze-dried, giving a dark green solid (19.92 mg). HPLC purity at 520 nm: 100%. MS: [M + H]⁺ 770.3.

### Example 3: Synthesis of Compound 3

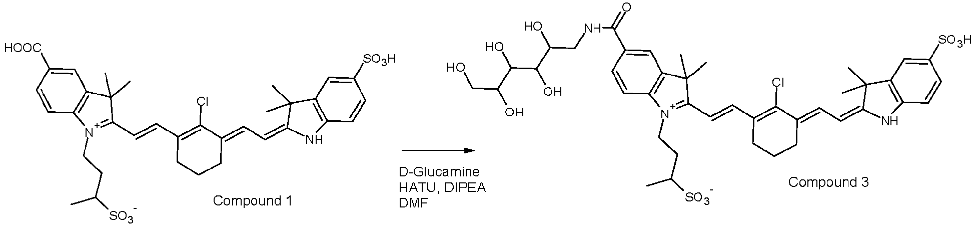

In a round bottom flask, Compound 1 (33.16 mg, 0.046 mmol) was dissolved in 5 mL of dry DMF. D-Glucamine (16.85 mg, 0.092 mmol), HATU (35.36 mg, 0.092 mmol) and DIPEA (32.1 µL, 0.184 mmol), were then added to the purple solution at RT. The mixture was stirred at RT for 2 hours. 50 mL of cold diethyl ether were added to the suspension and the precipitate was filtered. The purple solid was then purified by flash chromatography on a pre-packed C18 silica column (Biotage^{®} SNAP ULTRA, 30 g) with a milliQ water-acetonitrile gradient. Fractions containing the pure product were combined and concentrated under reduced pressure. The powder was re-dissolved in milliQ water and the solution was freeze-dried, giving a purple solid (35.0 mg, 86.6% yield). HPLC purity at 510 nm and 254 nm: 100%. MS: [M + H]⁺ 878.

### Example 4: Synthesis of Compound 4

### Preparation of intermediate (7)

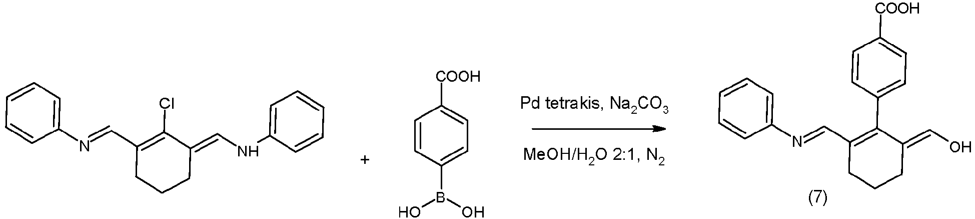

In a round bottom flask, dried under nitrogen atmosphere, N-[(3-(anilinomethylene)-2-chloro-1-cyclohexen-1-yl)methylene]aniline monohydrochloride (72.7 mg, 0.202 mmol) was dissolved in 50 mL of a degassed mixture of H₂O/MeOH 1:2. 4-Carboxyphenylboronic acid (60.4 mg, 0.364 mmol), Pd tetrakis (35.0 mg, 0.0303 mmol) and Na₂CO₃ (38.5 mg, 0.364 mmol) were added to the yellow solution at RT. The mixture was then heated at 80°C and stirred under nitrogen flow for 2 hours. The solvent was distilled under reduced pressure and the crude was purified by flash chromatography on a pre-packed C18 silica column (Biotage^{®} SNAP ULTRA, 30 g) with a milliQ water-acetonitrile gradient. Fractions containing intermediate (7) were combined and concentrated under reduced pressure. The powder was re-dissolved in milliQ water and the solution was freeze-dried, giving a bright orange solid (50.7 mg, 75.3% yield). HPLC purity at 410 nm: 100%. MS: [M + H]⁺ 334.1.

### Preparation of intermediate (8)

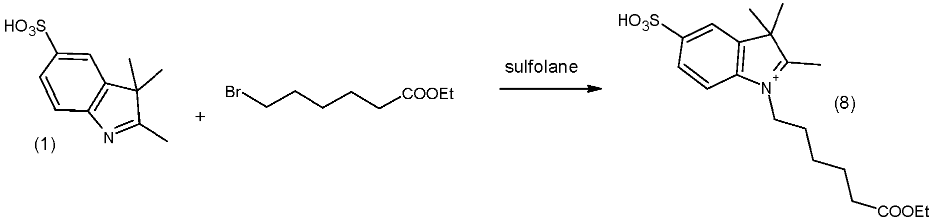

In a pressure reactor vessel, the intermediate (1) (1.02 g, 4.26 mmol) was suspended in 5 mL of sulfolane; ethyl 6-bromohexanoate (1.14 ml, 6.39 mmol) was then added to the suspension at RT. The brown suspension was then heated at 90°C for 70 hours. Cold ethyl acetate (10 mL) were added to the suspension and the precipitate was filtered. The red solid was then purified by flash chromatography on a pre-packed C18 silica column (Biotage^{®} SNAP, 120 g) with a milliQ water-acetonitrile gradient. Fractions containing Compound 3 were combined and concentrated under reduced pressure. The powder was re-dissolved in milliQ water and the solution was freeze-dried, giving a red solid (285 mg, 17.5% yield). HPLC purity at 270 and 254 nm: 100%. MS: [M + H]⁺ 382.1.

### Preparation of intermediate (9)

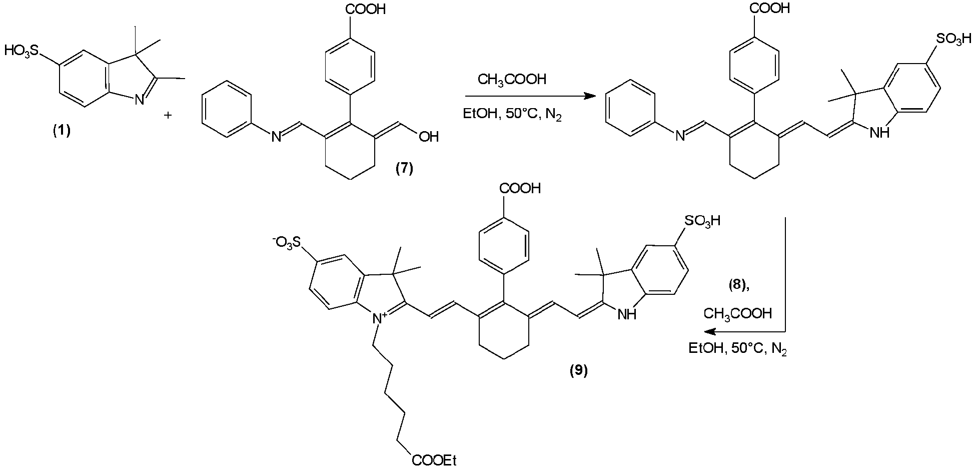

In a round bottom flask, dried under nitrogen atmosphere, the intermediate (7) (20.1 mg, 0.0602 mmol) was dissolved in 2 mL of absolute degassed ethanol. Then, a solution of intermediate (1) (14.4 mg, 0.0602 mmol) in 2 mL of absolute EtOH and 20 µL of acetic acid were added dropwise to the yellow solution at 50°C under nitrogen flow. The yellow solution became red after few minutes of heating then light brown as the temperature increased. After 4 hours the intermediate (8) (22.9 mg, 0.0602 mmol) was added to the solution. The solution was stirred at 50°C for 96 hours. The solvent was distilled under reduced pressure and the crude was purified by flash chromatography on a pre-packed C18 silica column (Biotage^{®} SNAP ULTRA, 30 g) with a milliQ water-acetonitrile gradient. Fractions containing intermediate (9) were combined and concentrated under reduced pressure. The powder was re-dissolved in milliQ water and the solution was freeze-dried, giving a purple-blue solid (8.2 mg, 16% yield). HPLC purity at 510 nm: 98.7%. MS: [M + H]⁺ 843.2.

### Preparation of intermediate (10)

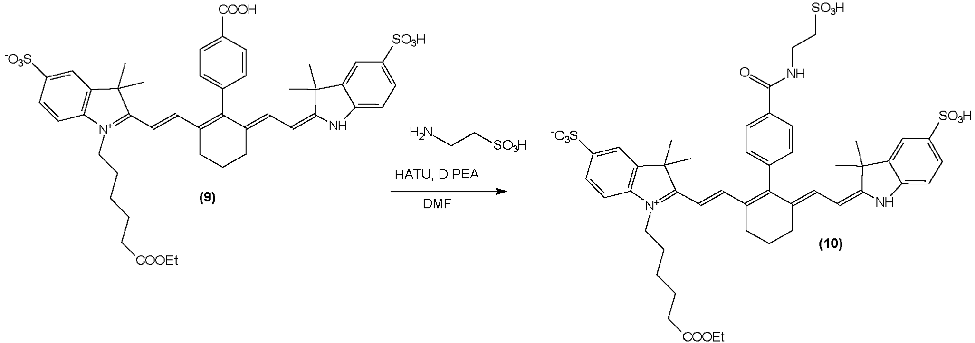

In a round bottom flask, the intermediate (9) (4 mg, 0.00474 mmol) was dissolved in 1.5 mL of dry DMF at RT. Then taurine, HATU and DIPEA were added to the purple solution. The mixture was stirred for 2 hours at RT under nitrogen atmosphere. Cold diethyl ether (20 mL) was added to the mixture. The precipitate was filtered, re-dissolved in milliQ water and purified on a pre-packed C18 silica column (Redisep Gold, 5 g) with a milliQ water-acetonitrile gradient. Fractions containing intermediate (10) were combined, concentrated under reduced pressure and freeze-dried giving a purple-blue solid (3.5 mg, 78% yield). HPLC purity at 510 nm: 98.4%, at 780 nm: 75%. MS: [M + H]⁺ 950.1.

### Synthesis of Compound 4

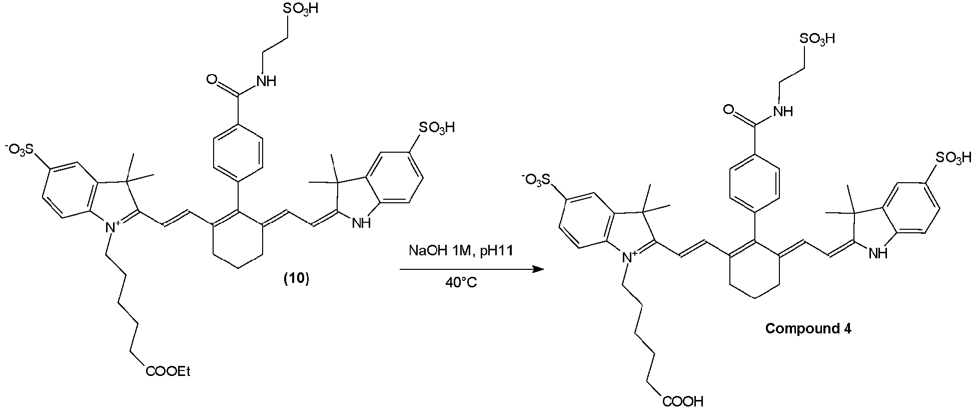

In a round bottom flask, the intermediate (10) (3.5 mg, 0.00368 mmol) was dissolved in 5 mL of milliQ water; the pH was adjusted to 11 adding NaOH 1M. The hydrolysis was performed at pH 11 and 40°C by automatic addition of NaOH 1M (Dosimat coupled with a pH-meter). After 2 hours HCl 1M was added adjusting the pH to 7. The mixture was desalted on a pre-packed C18 silica cartridge (Redisep Gold, 5 g) using as eluents: H₂O (10 CV) to remove NaCl and MeOH (5 CV) to recover the product. The eluate was distilled under reduced pressure and freeze-dried giving a purple-blue solid (2.6 mg, 76% yield). HPLC purity at 510 nm: 100%. MS: [M + H]⁺ 922.1.

### Example 5: Synthesis of Compound 5

### Preparation of intermediate (11)

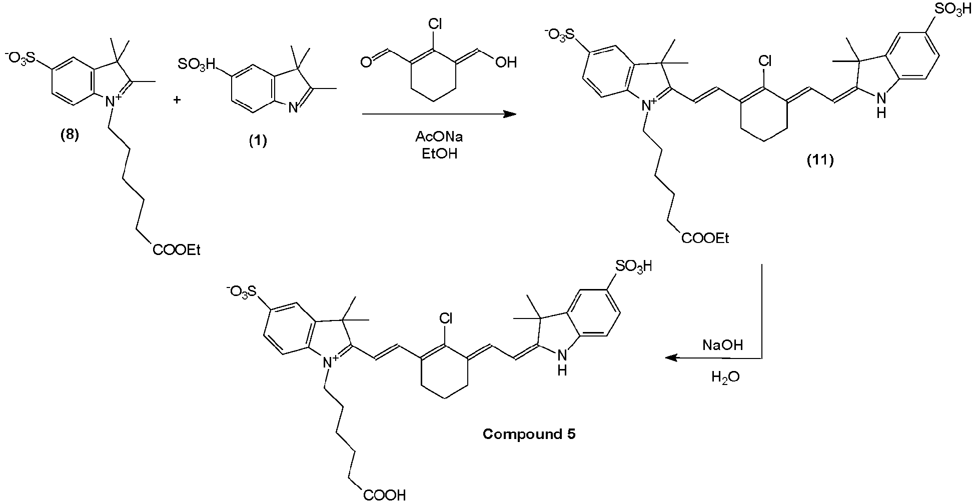

In a round bottom flask, dried under nitrogen atmosphere, the intermediate (8) (110.5 mg, 0.290 mmol) and intermediate (1) (90.0 mg, 0.377 mmol) were dissolved in EtOH (20 mL) at 50°C. Then, AcONa (23.8 mg, 0.290 mmol) and 2-chloro-3-(hydroxymethylene)-1-cyclohexene-1-carboxaldehyde (50.0 mg, 0.290 mmol) were added. The mixture was then heated at 50°C and stirred under nitrogen flow for 20 h. The solvent was dried under vacuum and the obtained solid was purified on a pre-packed C18 silica column (Biotage^{®} SNAP, 30 g) with a milliQ water-acetonitrile gradient. Fractions containing the pure product were combined, distilled under vacuum and freeze-dried, giving a dark violet solid (57.65 mg, 21.9% yield). HPLC purity at 510 nm: 99.9%, at 254 nm 93.3%. MS: [M + H]⁺ 757.35.

### Synthesis of Compound 5

Intermediate (11) (57.65 mg, 0.076 mmol) was dissolved in 40 mL of H₂O. The solution was brought at pH 11 with 0.1M NaOH (0.548 mL) and heated at 40°C, maintaining pH constant at 11 through automated addition of 0.1M NaOH with a Dosimat coupled with a pHmeter. The reaction was stopped after 8 h and 6.626 mL of NaOH were added. The mixture was cooled to room temperature, neutralized with 1M HCl (1.0 mL) and distilled under vacuum. The crude product was subsequently purified on a pre-packed C18 silica column (Biotage^{®} SNAP, 60 g) with a milliQ water-acetonitrile gradient. Fractions containing the pure product were combined, distilled under vacuum and freeze-dried, giving a dark green solid (31.9 mg, 57.5% yield). HPLC purity at 510 nm: 99.9%, at 254 nm 96.7%. MS: [M + H]⁺ 729.3.

### Example 6: Synthesis of Compound 6

### Preparation of intermediate (13)

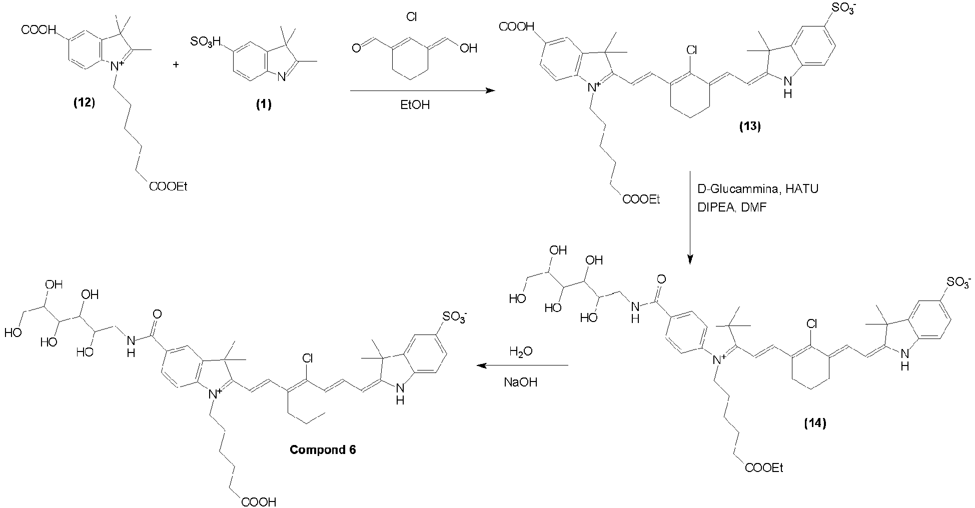

In a round bottom flask, dried under nitrogen atmosphere, intermediate (1) (90.0 mg, 0.377 mmol) was dissolved in EtOH (20 mL) at 50°C and then 2-chloro-3-(hydroxymethylene)-1-cyclohexene-1-carboxaldehyde (50.0 mg, 0.290 mmol) was added. The mixture was then heated at 50°C and stirred under nitrogen flow for 1.5 h. The intermediate (12) (100.3 mg, 0.290 mmol) was added to the mixture that was heated at 50°C and stirred under nitrogen flow for additional 13.5 h. Then, other intermediate (12) (30.1 mg, 0.087 mmol) was added and the reaction was stopped after 9 h of heating. The crude mixture was dried under vacuum (182.0 mg) and used in the following step with no further purification.

### Preparation of intermediate (14)

In a round bottom flask, crude intermediate (13) (182.0 mg, theoretically 0.252 mmol) was dissolved in 5 mL of dry DMF. D-glucamine (91.3 mg, 0.504 mmol), HATU (191.6 mg, 0.504 mmol) and DIPEA (158.0 µL, 0.907 mmol), were then added to the solution at RT. The mixture was stirred at RT for 4 h, then cold diethyl ether (70 mL) was added. The dispersion was kept under stirring for 12 h, the solvent was decanted and the obtained solid was washed with cold diethyl ether and purified on a pre-packed C18 silica column (Biotage^{®} SNAP, 60 g) with a milliQ water-acetonitrile gradient. Fractions containing the pure product were combined, distilled under vacuum and freeze-dried, giving a dark green solid (114.0 mg, 44% yield from intermediate (12)). HPLC purity at 510 nm: 99.4%, at 756 nm 40.0%. MS: [M + H]⁺ 884.48.

### Synthesis of Compound 6

Intermediate (14) (114.0 mg, 0.129 mmol) was dissolved in 60 mL of H₂O with the addition of 1 mL of EtOH. The solution was brought at pH 11 with 1M NaOH (0.432 mL) and heated at 40°C, maintaining pH constant at 11 through automated addition of 1M NaOH with a Dosimat coupled with a pHmeter. The hydrolysis was completed after 4.5 h and 6.626 mL of NaOH were added. The mixture was cooled to room temperature, neutralized with 2M HCl (1.5 mL) and distilled under vacuum. The crude product was subsequently purified on a pre-packed C18 silica column (Biotage^{®} SNAP, 60 g) with a milliQ water-acetonitrile gradient. Fractions containing the pure product were combined, distilled under vacuum and freeze-dried, giving a dark green solid (70.62 mg, 64% yield). HPLC purity at 510 nm: 99.4%, at 254 nm 93.9.0%. MS: [M + H]⁺ 856.90.

### Example 7: Synthesis of Compound 7

### Preparation of intermediate (15)

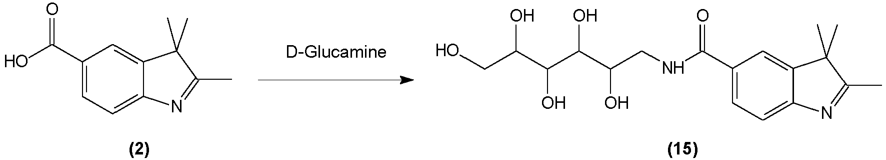

In a round bottom flask the intermediate (2) (1.01 g, 4.97 mmol) was dissolved in 15 mL of anhydrous DMF, then TBTU (2.23 g, 6.94 mmol) and DIPEA (1.73 mL, 9.95 mmol) were added. Consequently, D-glucamine (1.260 mg, 6.95 mmol) was added and the mixture was stirred for 2.5 hours at room temperature. Addition of cold diethyl ether allowed the precipitation of a solid that was filtered, dissolved in MeOH and dried under vacuum before the purification by flash chromatography on a pre-packed C18 silica column (Biotage^{®} SNAP Ultra 120 g) with a water-acetonitrile gradient. Fractions containing the pure product were combined and concentrated under vacuum. The batch was freeze-dried and a white solid was obtained (1.75 g, 95.9% yield). HPLC-MS purity at 270 nm: 100%; MS: [M + H]⁺ 367.0.

### Preparation of intermediate (16)

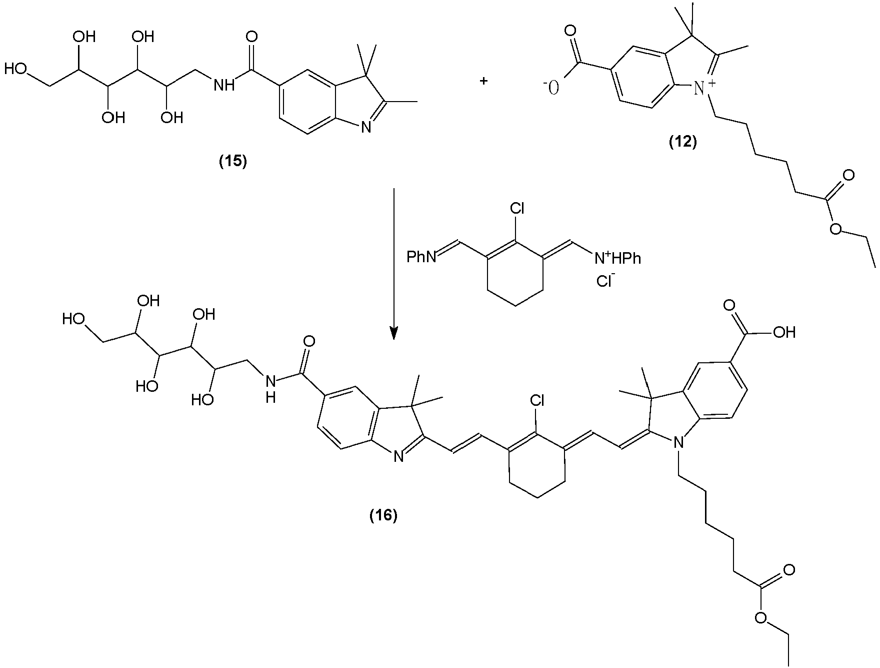

In a round bottom flask the intermediate (15) (132.56 mg, 0.3618 mmol) was dissolved in 45 mL of absolute EtOH and N-[(3-(anilinomethylene)-2-chloro-1-cyclohexen-1-yl)methylene]aniline monohydrochloride (100 mg, 0.2783 mmol) was added. The solution was heated at 50°C for 4 hours. Then, the intermediate (12) (96.41 mg, 0.2783 mmol) was added dissolved in 15 mL of absolute EtOH. The mixture was heated at 50°C for 24 hours. A second addition of intermediate (12) (48.58 mg, 0.1402 mmol) was made in order to achieve the highest conversion towards the asymmetric cyanine and the solution was stirred for additional 48 hours. The mixture was cooled to room temperature and distilled under vacuum, obtaining crude intermediate (16) that was used in the following reaction with no further purification. HPLC purity at 510 nm: 59.9%. MS: [M + H]⁺ 848.4.

### Preparation of intermediate (17)

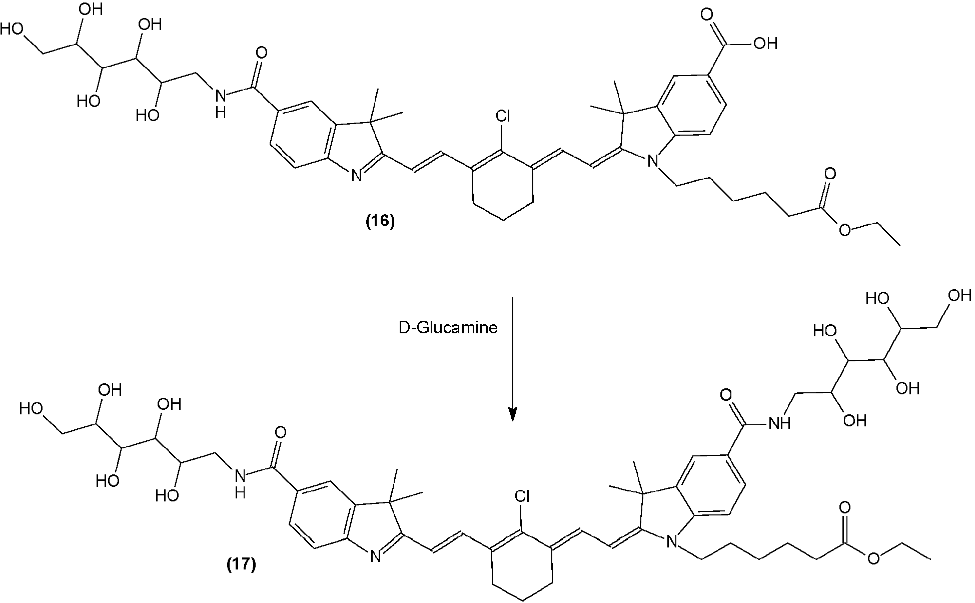

In a round bottom flask crude intermediate (16) (246 mg, 0.290 mmol theoretically) was dissolved in 20 mL of anhydrous DMF, then HATU (220.2 mg, 0.579 mmol) and DIPEA (181 µL, 1.042 mmol) were added. Consequently, D-glucamine (105 mg, 0.579 mmol) was added and the mixture was stirred for 4 hours at RT. The addition of cold diethyl ether caused the precipitation of a purple solid, that was filtered and washed twice with cold solvent and then purified by flash chromatography on a pre-packed C18 silica column (Biotage^{®} SNAP Ultra 120 g) with a water-acetonitrile gradient. Fractions containing the pure product were combined, distilled under reduced pressure and freeze-dried, affording a purple solid (70.0 mg, 25% yield from Compound 2). HPLC-MS purity at 510 nm: 100%; MS: [M + H]⁺ 1011.4.

### Synthesis of Compound 7

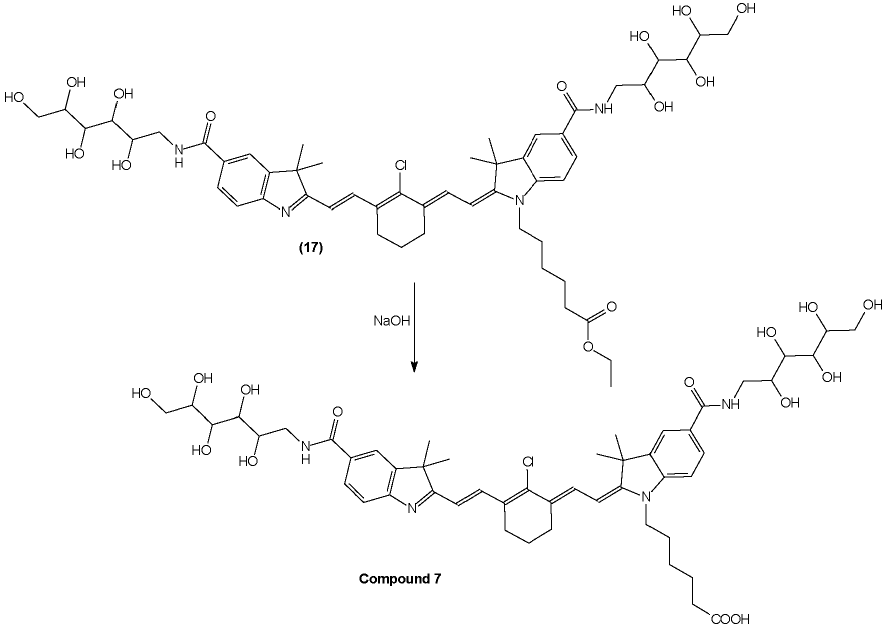

The intermediate (17) (30.00 mg, 0.0296 mmol) was dissolved in 15 mL of a solution H₂O/EtOH 7:3. The solution was brought at pH 11 with NaOH 1M and heated at 40°C, maintaining the pH constant at 11 through automated addition of NaOH 1M with a Dosimat coupled with a pHmeter. The hydrolysis was completed after 2 hours and 0.542 mL of NaOH 1M were added. The mixture was cooled to room temperature, neutralized with HCl 0.1M and distilled under vacuum, giving the crude product that was subsequently purified by flash chromatography on a pre-packed C18 silica column (Biotage^{®} SNAP Ultra 30 g) with a water-acetonitrile gradient. Fractions containing the pure product were combined, concentrated under vacuum and freeze-dried obtaining a purple solid (15.0 mg, 51.5% yield). HPLC-MS purity at 510 nm: 100%; MS: [M + H]⁺ 983.3.

### Example 10: Synthesis of Compound 10

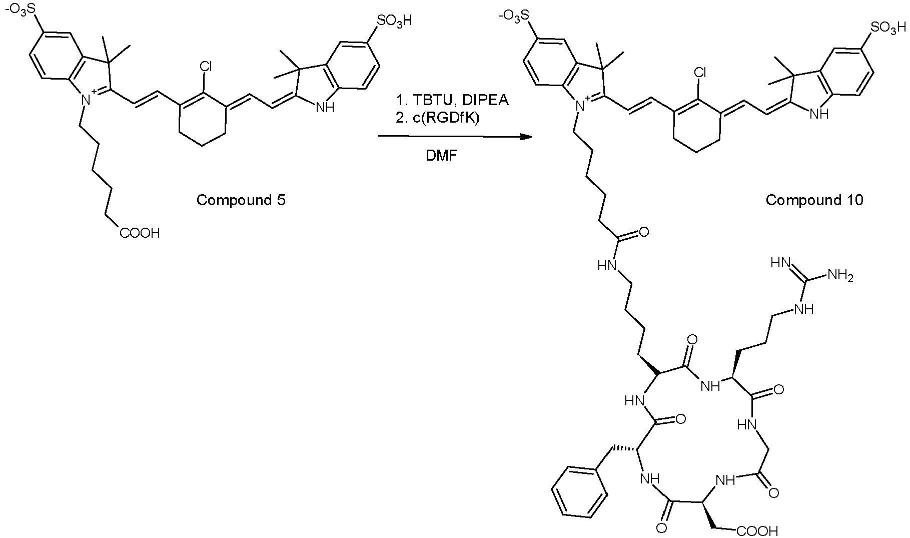

In a round bottom flask under nitrogen atmosphere Compound 5 (10 mg, 0.0137 mmol) was dissolved in 5 mL of dry DMF. TBTU (4.4 mg, 0.0137 mmol) and DIPEA (6.7 µL, 0.0383 mmol) were then added. The mixture was stirred for 1 hour at RT, then a solution of c(RGDfK) trifluoroacetate (9.8 mg, 0.0137 mmol) in 5 mL of dry DMF was dropped. The mixture was stirred at RT overnight, then it was precipitated with cold diethyl ether (150 mL) in an ice bath. The purple precipitate was filtered and washed twice with cold diethyl ether. It was then dissolved in a mixture of water/acetonitrile 2:1 and purified by flash chromatography on a pre-packed C18 silica column (Biotage^{®} SNAP Ultra 12 g) with a water-acetonitrile gradient. Fractions containing the pure product were combined, concentrated under reduced pressure and freeze-dried, giving a blue solid (9 mg, 50% yield). HPLC purity at 510 nm: 99.3%; MS: [M - H]⁻ 1314.3.

### Example 11: Synthesis of Compound 11

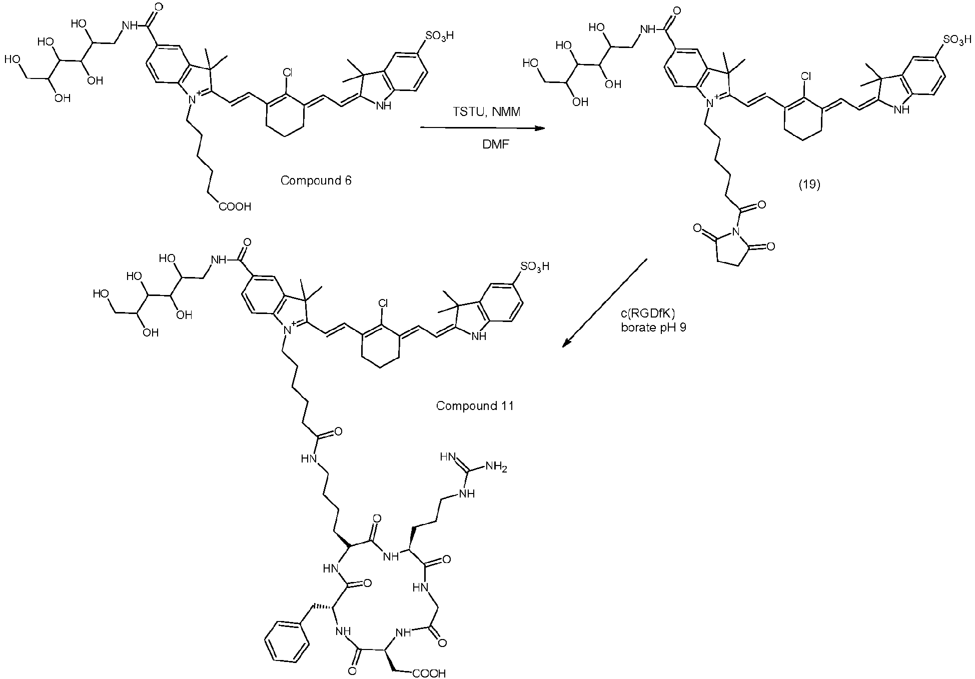

### Preparation of intermediate (19)

In a 40 mL centrifuge glass tube, Compound 6 (15.60 mg, 0.018 mmol) was dissolved in 3 mL of dry DMF. TSTU (10.9 mg, 0.036 mmol) and NMM (4 µL, 0.036 mmol) were then added to the purple solution at RT. The mixture was stirred at RT for 2 hours. Then, cold diethyl ether (40 mL) was added to the solution. The suspension was centrifuged at 4000 rpm for 55 min. Then, diethyl ether was decanted affording a purple solid stuck on the tube walls. The crude compound was used in the following step with no further purification.

### Synthesis of Compound 11

In the same 40 mL centrifuge glass tube containing intermediate (19), a solution of c(RGDfK) (14 mg, 0.020 mmol) in 8 mL of borate buffer at pH 9 was added at RT. The purple mixture was stirred at RT for 2 hours. Then, the pH of the mixture was adjusted to 7 by addition of 1M HCl and the crude was purified by preparative HPLC with a 0.1% formic acid/acetonitrile gradient on a YMC triart-phenyl column. Fractions containing the pure product were combined, concentrated and desalted on a pre-packed silica C18 column (GE, 5 g) using 10 CV of water to remove the excess of formic acid and 5 CV of MeOH to recover the product. The final solution was then distilled under reduced pressure, the resulting powder was dissolved in milliQ water and the solution was freeze-dried, giving a bright purple solid (6.5 mg, 25% yield). HPLC purity at 510 nm: 100%, at 254 nm 89.3%. MS: [M + H]⁺ 1441.4.

### Example 12: Synthesis of Compound 12

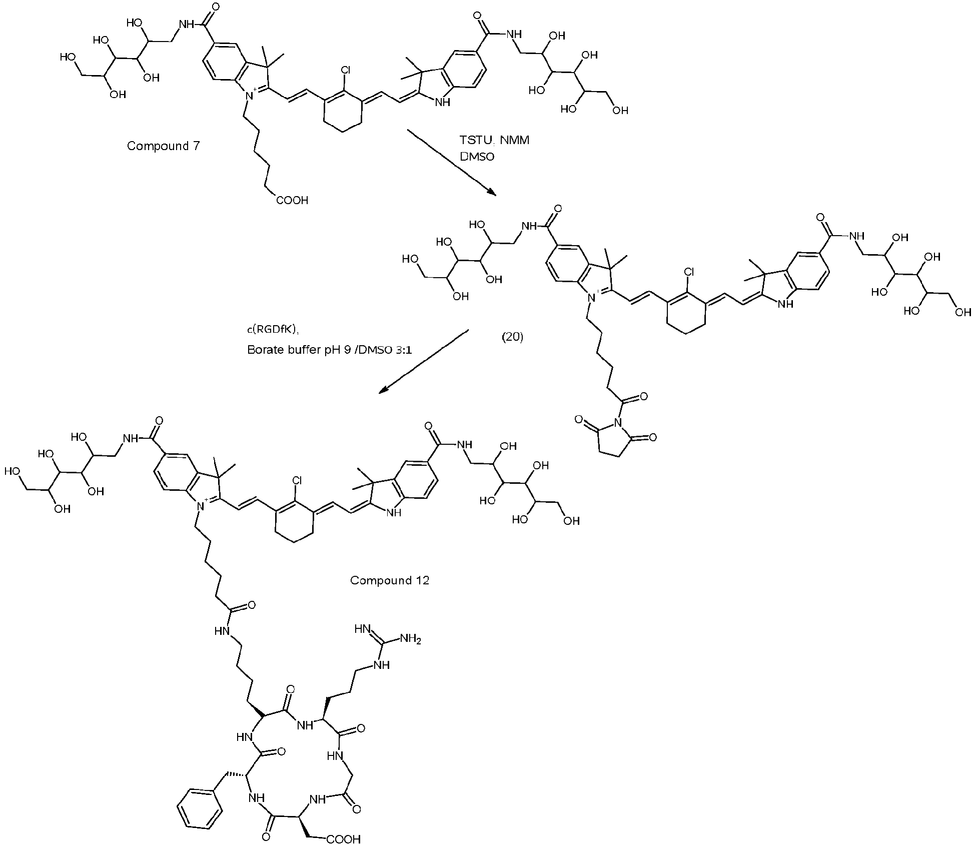

### Preparation of intermediate (20)

In a 40 mL glass centrifuge tube, Compound 7 (10.3 mg, 0.0105 mmol) was dissolved in 2 mL of dry DMSO. TSTU (6.3 mg, 0.0210 mmol) and NMM (2.3 µL, 0.0210 mmol) were then added to the purple solution at RT. The mixture was stirred at RT for 30 minutes. Then, 40 mL of cold diethyl ether were added to the solution. The suspension was centrifuged at 4000 rpm for 55 min and diethyl ether was decanted, affording a purple solid stuck on the tube walls. The crude was used in the following reaction with no further purification.

### Synthesis of Compound 12

In the same 40 mL centrifuge tube, the intermediate (20) was dissolved in 2 mL of DMSO and a solution of c(RGDfK) (8.3 mg, 0.0116 mmol) in 6 mL of borate buffer pH 9 was added at RT. The purple mixture was then stirred at RT for 1 hour. The pH of the mixture was adjusted to 6.7 by addition of 0.5M HCl. Water was distilled under reduced pressure and the crude was purified by flash chromatography on a pre-packed C18 silica column (Phenomenex^{®} CLARICEP, 40 g) with a milliQ water-acetonitrile gradient. Fractions containing the pure product were combined and concentrated under reduced pressure. The powder was re-dissolved in milliQ water and the solution was freeze-dried, giving a bright purple solid (4.6 mg, 27.9% yield from compound 7). HPLC purity at 510 nm: 97.8%. MS: [M + H]⁺ 1569.5.

### Example 13: Optical properties

### Analytical characterization of the dyes

For each compound an analytical and optical characterization was performed. All the dyes synthesized, and conjugates thereof, were extensively characterized by HPLC-MS, achieving high values of final purity. For instance, the purity calculated from the HPLC area at 254 nm, 510 nm (maximum absorbance at neutral pH) and 780 nm of the representative Compound 5 is reported in the following Table II

**Table II - Purity % (HPLC area) of the representative Compound 5 at different wavelengths.**

| **Purity % (HPLC Area)** | **254 nm** | **510 nm** | **780 nm** |
|---|---|---|---|
| Compound 5 | >95% | >98% | >95% |

Optical characterization was performed for each compound with Spark^{®} multimode microplate reader, registering with specific 96-wells plate the absorbance, excitation and emission spectra of the fluorophore buffered in phosphate buffer at several pH, ranging from 3 to 8.

In particular, the absorbance, excitation and emission maxima of representative compounds of formula (I) and conjugates of formula (II) are shown in Table III.

**Table III - Optical characterization of the pH responsive dyes and conjugates**

| **Dyes of formula (I)** | **Abs. Max. (nm)** | **Exc. Max. (nm)** | **Em. Max. (nm)** |
|---|---|---|---|
| Compound 1 | 784 | 783 | 804 |
| Compound 2 | 783 | 780 | 803 |
| Compound 3 | 782 | 780 | 807 |
| Compound 4 | 763 | 758 | 788 |
| Compound 5 | 780 | 780 | 804 |
| Compound 6 | 784 | 784 | 805 |
| Compound 7 | 789 | 786 | 808 |

| **Conjugates of formula (II)** | **Abs. Max. (nm)** | **Exc. Max. (nm)** | **Em. Max. (nm)** |
|---|---|---|---|
| Compound 10 | 784 | 780 | 804 |
| Compound 11 | 789 | 788 | 808 |
| Compound 12 | 793 | 790 | 814 |

| | | | |
|---|---|---|---|
| n/a, not available. | | | |

The compounds of the invention are characterized by absorption maxima comprised in the range from about 620 nm to about 790 nm, with fluorescence emission in the range from about 650 nm to 900 nm even when conjugated to a targeting moiety.

### pK assessment

From the analysis of the variation of the absorbance and emission with respect to the variation of pH, it was possible to calculate the pKa of the dyes of the invention as the inflection point of the interpolated curve.

All the pH buffers used for the experiment were phosphate based buffers and each pH point has been accurately checked with pH-meter. First the cyanine powder was dissolved in water at ca. 1 mg/mL concentration and then 10 µL of this mother solution were added to 1 ml of phosphate buffers at different pH, previously prepared mixing ortophosphoric acid and sodium hydroxide in the right amount to reach the desired pH. The diluted cyanine buffered solutions were then sampled and transferred into a 96 wells plate (clear for the absorbance measurements and dark for the emission measurements) for the analyses. By plotting for each solution the value of the absorbance at the maximum of the active form, for instance 780 nm for Compound 5, vs. the pH of the solution, a polynomial cubic curve was obtained, whose flex corresponds to the pKa value of the cyanine. An example of such curve is shown in Figure 1 for the representative Compound 5, where the inflection point has provided a pKa value of 5.9.

Moreover, a similar curve was obtained plotting the value of emission at the maximum of the active form, for instance at 804 nm for Compound 5, vs. pH, confirming the same value of pKa (result obtained from a second source of data). The pKa value obtained for the Reference Compound A is also displayed in Table IV.

**Table IV - pK values of representative compounds of the invention**

| **Dyes of formula (I)** | **pKa (by abs)** |
|---|---|
| ***Reference Comp. A**** | ***4.6*** |
| Compound 1 | 6.0 |
| Compound 4 | 6.4 |
| Compound 5 | 5.9 |
| Compound 6 | 6.0 |
| Compound 7 | 6.1 |

| **Conjugates of formula (II)** | **pKa (by abs)** |
|---|---|
| Compound 10 | 5.8 |
| Compound 11 | 6.3 |

| | |
|---|---|
| *Lee et al., Bioconjug Chem 2011; 22(4): 777-784 | |

### Residual fluorescence at different pH

The ratio of the fluorescence at different pH (5, 6 and 7.5) with respect to the maximum fluorescence collected at pH 3 (highly acidic) was calculated for several representative compounds of the invention and the results are reported in the following Table V. The results were also compared to the values calculated for the non-alkylated Reference Compound A.

**Table V - Residual fluorescence at different pH**

| **Compound** | **pKa** | **Em. at pH 5/ Em at pH 3** | **Em. at pH 6/ Em at pH 3** | **Em. at pH 7.5/ Em at pH 3** |
|---|---|---|---|---|
| ***Reference Compound A**** | ***4.6*** | ***40*** | ***10*** | ***1*** |
| Compound 1 | 6.0 | 104 | 55 | 9 |
| Compound 4 | 6.4 | 108 | 80 | 19 |
| Compound 5 | 5.9 | 89 | 55 | 5 |

| | | | | |
|---|---|---|---|---|
| *Lee et al., Bioconjug Chem 2011; 22(4): 777-784 | | | | |

These results demonstrate that the higher pKa of the present compounds, comprised in the range of 5.5-7.0, in particular 5.8-6.5, allows to improve the fluorescence emission at pH 5 and 6, i.e. at the pH values more likely characterizing the tumoral extracellular environment and the cellular endosomes, and to provide an easier detectable signal in vitro and in vivo. For instance, at pH 5 and 6 they displayed a fluorescence signal from about 2 to 8 times higher than the Reference Compound A. On the contrary, the fluorescence at physiologycal pH (about 7.4) is very low, which may result in low background signal.

### References:

1. Cherrick et al., J Clin Invest 1960;39(4): 592-600
2. Tummers Q. et al., PlosOne 2015;10(6): e0129766
3. Achilefu S. et al, J Med Chem 2002; 45, 2003-2015
4. Fidel J. et al., Cancer Res. 2015;15; 75(20): 4283-4291
5. Damaghi M. et al., Front Physiol. 2013; 4:370
6. WO00/75237
7. Briggs M. et al., Chem. Commun. 2000, 2323-2324
8. WO2004/039894
9. Beletskii A. et al., BioTechniques 2005, 39:894-897
10. Lee H. et al., Bioconjug Chem. 2011, 22(4): 777-784
11. Gilson R. et al., Mol. Pharmaceutics 2015, 12: 4237-4246
12. T.W. Green and P.G.M.Wuts, Protective Groups in Organic Synthesis, Wiley, N.Y. 2007, 4th Ed., Ch. 5
13. Kapp et al., Sci Rep, 2017, 7:3905
14. Wichert et al., Nat Chem 2015, 7: 241-249
15. Li et al., FASEB J 2005, 19:1978-85
16. Williams et al., Chem Biol Drug Des 2018, 91:605-619
17. Mujumdar R.B. et al., Bioconjugate Chem. 1993, 4(2): 105-111
18. Yamana et al, Chem. Pharm. Bull., 1972, 20(5), 881-891
19. US 7,408,062 B2
20. US 2013/045488 A1

## Claims

1. A compound of formula (I), or a pharmaceutically acceptable salt thereof, 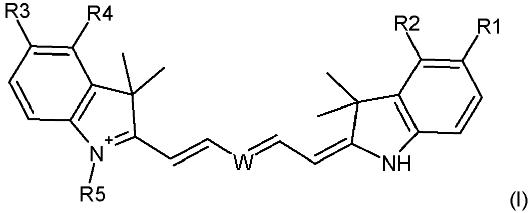 wherein
W is a group 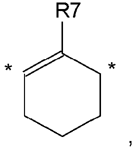 where
R7 is chlorine or phenyl optionally substituted by a group selected from -SO₃H, -COOH, -CONH-Y, -alkyl-COOH and -alkyl-CONH-Y, where
Y is a bivalent alkyl substituted by -SO₃H or at least two hydroxyl groups, and
* represents a bonding position;
R1 and R3 are independently selected from the group consisting of hydrogen, -SO₃H, - COOH and -CONH-Y; and R2 and R4 are hydrogen;
R5 is an alkyl optionally substituted by a group selected from -SO₃H, -COOH and -CONH₂.

2. A conjugate of a compound (I) as defined in claim 1 represented by a compound of formula (II) 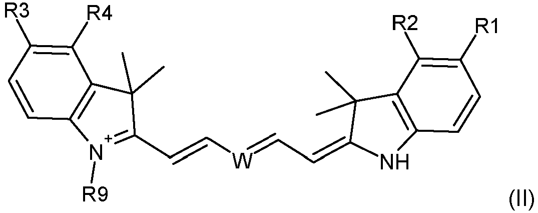 wherein
W is a group 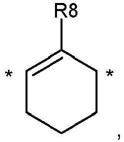 where
R8 is chlorine or phenyl optionally substituted by a group selected from -SO₃H, -COOH, CONH-Y, -alkyl-COOH, -alkyl-CONH-Y or -R10, where
Y is a bivalent alkyl substituted by -SO₃H or at least two hydroxyl groups,
R10 is a bivalent alkyl substituted by a group -CONH-(S)ₘ-T, where S is a spacer selected from -(CH₂)ₚCOO-, -(CH₂CH₂O)ₚCH₂CH₂COO- and - (CH₂CH₂O)ₚCH₂CH₂NH-, wherein p is an integer between 0 and 20;
T is a targeting moiety selected from the group consisting of a small molecule, a protein, a peptide, a peptidomimetic, an enzyme substrate, an antibody or fragment thereof and an aptamer; and
m is an integer equal to 0 or 1; and
* represents a bonding position;
R1 and R3 are independently selected from the group consisting of hydrogen, -SO₃H, -COOH and -CONH-Y; and R2 and R4 are hydrogen;
R9 is an alkyl optionally substituted by a group selected from -SO₃H, -COOH, -CONH₂, and -CONH-(S)ₘ-T, wherein S, T and m are defined above;
and wherein at least one group -CONH-(S)ₘ-T is present in R9 or R10.

3. The compound of formula (II) according to claim 2, wherein T is a moiety interacting with an integrin receptor.

4. A compound of formula (I) as defined in claim 1 or a compound of formula (II) as defined in claim 2-3 for use as fluorescent probe for biomedical optical imaging applications in mammals.

5. The compound of formula (I) or (II) for use according to claim 4, wherein the imaging applications are directed to the detection of abnormal tissues, including a primary tumor lesion, local or distant metastases, or a pre-neoplastic lesion, and are carried out under NIR radiation.

6. A pharmaceutical composition comprising the compound of formula (I) as defined in claim 1 or the compound of formula (II) as defined in claims 2-3 and at least one pharmaceutically acceptable carrier or excipient.

7. Diagnostic kit comprising at least one compound of formula (I) as defined in claim 1 or at least one compound of formula (II) as defined in claims 2-3 together with additional adjuvants thereof for implementing the biomedical optical imaging applications.

8. A compound of formula (I) as defined in claim 1 or a compound of formula (II) as defined in claims 2-3 for use in a method of imaging tissues and cells comprising the steps of:
i) contacting the cells or tissues with a compound of formula (I) as defined in claim 1 or a compound of formula (II) as defined in claims 2-3;
ii) irradiating the tissues or cells at a wavelenght absorbed by the imaging agent;
iii) detecting the near-infrared emission using a fluorescence camera.

## Patentansprüche

1. Verbindung der Formel (I) oder pharmazeutisch unbedenkliches Salz davon, 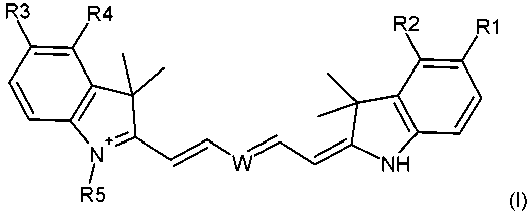 wobei
W für eine Gruppe 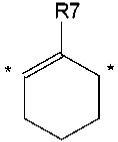 steht, wobei
R7 für Chlor oder Phenyl, das gegebenenfalls durch eine Gruppe, die aus -SO₃H, -COOH, -CONH-Y, -Alkyl-COOH und - Alkyl-CONH-Y ausgewählt ist, substituiert ist, wobei
Y für ein zweiwertiges Alkyl, das durch -SO₃H oder mindestens zwei Hydroxylgruppen substituiert ist, steht und
* für eine Bindungsposition steht;
R1 und R3 unabhängig aus der Gruppe bestehend aus Wasserstoff, -SO₃H, -COOH und -CONH-Y ausgewählt sind; und R2 und R4 für Wasserstoff stehen;
R5 für ein Alkyl, das gegebenenfalls durch eine Gruppe, die aus -SO₃H, -COOH und -CONH₂ ausgewählt ist, substituiert ist, steht.

2. Konjugat einer Verbindung (I) gemäß Anspruch 1, wiedergegeben durch eine Verbindung der Formel (II) 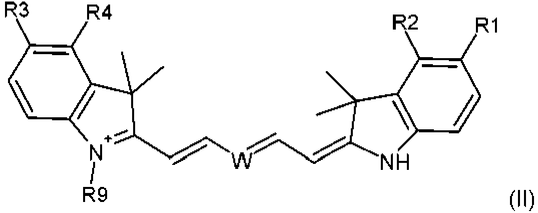 wobei
W für eine Gruppe 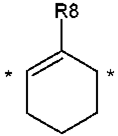 steht, wobei
R8 für Chlor oder Phenyl, das gegebenenfalls durch eine Gruppe, die aus -SO₃H, -COOH, -CONH-Y, -Alkyl-COOH, - Alkyl-CONH-Y oder -R10 ausgewählt ist, substituiert ist, wobei
Y für ein zweiwertiges Alkyl, das durch -SO₃H oder mindestens zwei Hydroxylgruppen substituiert ist, steht, R10 für ein zweiwertiges Alkyl, das durch eine Gruppe - CONH-(S)ₘ-T substituiert ist, steht, wobei
S für einen Spacer steht, der aus -(CH₂)ₚCOO-, - (CH₂CH₂O)ₚCH₂CH₂COO- und -(CH₂CH₂O)ₚCH₂CH₂NH- ausgewählt ist, wobei p für eine ganze Zahl zwischen 0 und 20 steht; T für eine Targeting-Gruppierung steht, die aus der Gruppe bestehend aus einem kleinen Molekül, einem Protein, einem Peptid, einem Peptidomimetikum, einem Enzymsubstrat, einem Antikörper oder Fragment davon und einem Aptamer ausgewählt ist; und
m für eine ganze Zahl mit einem Wert von 0 oder 1 steht; und
* für eine Bindungsposition steht;
R1 und R3 unabhängig aus der Gruppe bestehend aus Wasserstoff, -SO₃H, -COOH und -CONH-Y ausgewählt sind; und R2 und R4 für Wasserstoff stehen;
R9 für ein Alkyl, das gegebenenfalls durch eine Gruppe, die aus -SO₃H, -COOH, -CONH₂ und -CONH-(S)ₘ-T ausgewählt ist, substituiert ist, steht, wobei S, T und m oben definiert sind;
und wobei mindestens eine Gruppe -CONH-(S)ₘ-T in R9 oder R10 vorliegt.

3. Verbindung der Formel (II) nach Anspruch 2, wobei T für eine mit einem Integrinrezeptor wechselwirkende Gruppierung steht.

4. Verbindung der Formel (I) gemäß Anspruch 1 oder Verbindung der Formel (II) gemäß Anspruch 2-3 zur Verwendung als Fluoreszenzsonde für biomedizinische optische Bildgebungsanwendungen bei Säugetieren.

5. Verbindung der Formel (I) oder (II) zur Verwendung nach Anspruch 4, wobei die Bildgebungsanwendungen auf den Nachweis von abnormalen Geweben, einschließlich einer Primärtumorläsion, lokaler oder entfernter Metastasen oder einer präneoplastischen Läsion, gerichtet sind und unter NIR-Strahlung durchgeführt werden.

6. Pharmazeutische Zusammensetzung, umfassend die Verbindung der Formel (I) gemäß Anspruch 1 oder die Verbindung der Formel (II) gemäß den Ansprüchen 2-3 und mindestens einen pharmazeutisch unbedenklichen Träger oder Exzipienten.

7. Diagnostisches Kit, umfassend mindestens eine Verbindung der Formel (I) gemäß Anspruch 1 oder mindestens eine Verbindung der Formel (II) gemäß den Ansprüchen 2-3 zusammen mit zusätzlichen Adjuvantien davon zur Implementierung der biomedizinischen optischen Bildgebungsanwendungen.

8. Verbindung der Formel (I) gemäß Anspruch 1 oder Verbindung der Formel (II) gemäß den Ansprüchen 2-3 zur Verwendung bei einem Verfahren zur Bildgebung von Geweben und Zellen, umfassend die Schritte:
i) Inkontaktbringen der Zellen oder Gewebe mit einer Verbindung der Formel (I) gemäß Anspruch 1 oder einer Verbindung der Formel (II) gemäß den Ansprüchen 2-3;
ii) Bestrahlen der Gewebe oder Zellen mit einer Wellenlänge, die von dem Bildgebungsmittel absorbiert wird;
iii) Nachweisen der Nahinfrarotemission mit einer Fluoreszenzkamera.

## Revendications

1. Composé de formule (I), ou sel pharmaceutiquement acceptable de celui-ci, 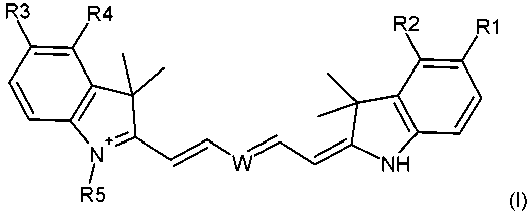 dans laquelle
W est un groupe 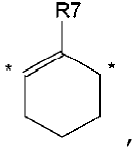 où
R7 est chlore ou phényle facultativement substitué par un groupe choisi parmi -SO₃H, -COOH, -CONH-Y, -alkyl-COOH et -alkyl-CONH-Y, où
Y est un alkyle bivalent substitué par -SO₃H ou au moins deux groupes hydroxyle, et
* représente une position de liaison ;
R1 et R3 sont choisis indépendamment dans le groupe constitué par hydrogène, -SO₃H, -COOH et -CONH-Y ; et R2 et R4 sont hydrogène ;
R5 est un alkyle facultativement substitué par un groupe choisi parmi -SO₃H, -COOH et -CONH₂.

2. Conjugué d'un composé (I) tel que défini dans la revendication 1 représenté par un composé de la formule (II) 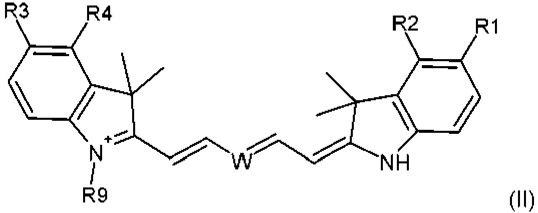 dans laquelle
W est un groupe 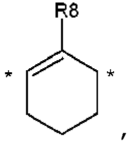 où
R8 est chlore ou phényle facultativement substitué par un groupe choisi parmi -SO₃H, -COOH, -CONH-Y, -alkyl-COOH, -alkyl-CONH-Y ou -R10, où
Y est un alkyle bivalent substitué par -SO₃H ou au moins deux groupes hydroxyle,
R10 est un alkyle bivalent substitué par un groupe -CONH-(S)ₘ-T, où
S est un espaceur choisi parmi -(CH₂)ₚCOO-, - (CH₂CH₂O)ₚCH₂CH₂COO- et -(CH₂CH₂O)ₚCH₂CH₂NH-, où p est un entier compris entre 0 et 20 ;
T est un groupement de ciblage choisi dans le groupe constitué par une petite molécule, une protéine, un peptide, un peptidomimétique, un substrat enzymatique, un anticorps ou un fragment de ce dernier et un aptamère ; et
m est un entier égal à 0 ou 1 ; et
* représente une position de liaison ;
R1 et R3 sont choisis indépendamment dans le groupe constitué par hydrogène, -SO₃H, -COOH et -CONH-Y ; et R2 et R4 sont hydrogène ;
R9 est un alkyle facultativement substitué par un groupe choisi parmi -SO₃H, -COOH, -CONH₂, et -CONH-(S)ₘ-T, où S, T et m sont définis ci-dessus ;
et où au moins un groupe -CONH-(S)ₘ-T est présent dans R9 ou R10.

3. Composé de la formule (II) selon la revendication 2, dans laquelle T est un groupement interagissant avec un récepteur d'intégrine.

4. Composé de formule (I) tel que défini dans la revendication 1 ou composé de formule (II) tel que défini dans la revendication 2-3 destiné à être utilisé en tant que sonde fluorescente pour des applications d'imagerie optique biomédicale chez des mammifères.

5. Composé de formule (I) ou (II) destiné à être utilisé selon la revendication 4, dans lequel les applications d'imagerie sont dirigées sur la détection de tissus anormaux, y compris une lésion tumorale primaire, des métastases locales ou distantes, ou une lésion pré-néoplasique, et sont réalisées sous rayonnement NIR.

6. Composition pharmaceutique comprenant le composé de formule (I) tel que défini dans la revendication 1 ou le composé de formule (II) tel que défini dans les revendications 2-3 et au moins un support ou excipient pharmaceutiquement acceptable.

7. Kit de diagnostic comprenant au moins un composé de formule (I) tel que défini dans la revendication 1 ou au moins un composé de formule (II) tel que défini dans les revendications 2-3 ainsi que des adjuvants supplémentaires de celui-ci pour la mise en œuvre des applications d'imagerie optique biomédicale.

8. Composé de formule (I) tel que défini selon la revendication 1 ou composé de formule (II) tel que défini selon les revendications 2-3 destiné à être utilisé dans un procédé d'imagerie de tissus et de cellules comprenant les étapes de :
i) mise en contact des cellules ou tissus avec un composé de formule (I) tel que défini dans la revendication 1 ou un composé de formule (II) tel que défini dans les revendications 2-3 ;
ii) irradiation des tissus ou cellules à une longueur d'onde absorbée par l'agent d'imagerie ;
iii) détection de l'émission proche infrarouge en utilisant une caméra de fluorescence.
